# EUROPEAN PATENT APPLICATION

(11) **EP 4 183 401 A1**
(43) Date of publication of application: **24.05.2023**
(21) Application number: 21383041.7
(22) Date of filing: 17.11.2021
(51) Int. Cl.: A61K 35/28, C12Q 1/6876

(54) **IN VITRO METHOD FOR THE IDENTIFICATION OF MESENCHYMAL STEM/STROMAL CELLS**

(71) Applicant: Fundación Instituto de Estudios Ciencias de la Salud de Castilla y León (IECSCYL-IBSAL), 37007 Salamanca (ES); Consejo Superior de Investigaciones Científicas, M.P (CSIC), 28006 Madrid (ES)
(72) Inventor: DE LAS RIVAS SANZ, Francisco Javier, 28006 Madrid (ES); SANCHEZ-GUIJO MARTIN, Fermín, 37007 Salamanca (ES); MUNTION OLAVE, María Sandra, 37007 Salamanca (ES); SANCHEZ LUIS, Elena, 37007 Salamanca (ES)
(74) Representative: Hoffmann Eitle

(57) **Abstract**

The present invention refers to an *in vitro* method for identifying mesenchymal stem/stromal cells in a cell population obtained from a subject and/or for differentiating mesenchymal stem/stromal cells from Fibroblasts. The present invention also refers to a substantially pure population of mesenchymal stem/stromal cells, which have been isolated following the method described in the present invention.

## Description

### FIELD OF THE INVENTION

The present invention pertains to the medical field. Particularly, the present invention refers to an *in vitro* method for identifying human Mesenchymal Stem/Stromal Cells (usually referred to as MSC) in a cell population obtained from a subject and/or for differentiating Mesenchymal Stem/Stromal Cells from FIBroblasts (FIB). The present invention also refers to a substantially pure population of Mesenchymal Stem/Stromal Cells, which have been isolated following the method described in the present invention.

### STATE OF THE ART

FIBroblasts (FIB) are present in the stromal component of every organ and are the main providers of the extracellular matrix, mainly collagen, that maintains their structure. Mesenchymal Stromal Cells (MSC), also known as Mesenchymal Stem Cells, constitute a non-hematopoietic population that reside in various cellular tissues, including bone marrow and adipose tissue. They have FIBroblastic appearance when adhere to plastic surfaces and are able to self-renewal, with multipotent capacity and, therefore, able to differentiate into a number of cell types, including osteoblasts, chondrocytes and adipocytes.

To define MSC, the International Society for Cell Therapy (ISCT) has established several minimum criteria. Currently, there is no individual biomolecular marker capable of uniquely identifying MSC. Some authors have suggested that MSC and FIB derive from a common ancestor and share most of their features. In this regard, both cell types show a great resemblance in terms of morphology, adherence to plastic and proliferation capacity. Regarding surface proteins it has been seen that so-called FIBroblast-specific markers are also present on MSC and vice versa. However, expression levels of these common markers may vary depending on the cell passage of the cells. In addition, both MSC and FIB in certain conditions seem to be able to differentiate into other cell types such as: osteoblasts, myoblasts, adipocytes and chondrocytes, although there is controversy with other cell lineages; it is also controversial to know if there is any FIB subtype that could be really multipotent. It has been suggested that the anti-inflammatory and angiogenic activity in MSC from adipose tissue (AT-MSC) is much higher than in FIBroblasts. There are also well reported differences in the methylation pattern, where FIBroblasts have less CpG sites than MSC.

Gene expression profiling (GEP) could be among the technologies able to differentiate between the two cell types. But most GEP data show that MSC and FIB share similar expression profiles, although with some relevant differences, mainly in transmembrane and tumor- and cancer-associated genes, and in the function and development of the stem cells. Nevertheless, although both cell types have similar properties, MSC are by far the main option chosen for clinical development, with up to 1,300 clinical trials registered in international databases assessing clinically the therapeutic potential of MSC.

Currently developed genome-wide omics technologies and, more specifically, transcriptomic profiling methods are very powerful tools for achieving in-depth characterization of the genes that are expressed and activated in each different cell type. In this work we have generated a complete transcriptome profiling of bone-marrow derived human MSC (BM-MSC), performing a robust analysis of three data sets that provide full quantification of the gene expression of different cell types (including MSC and FIB). Each data set is independent and has been produced with different transcriptomic platforms: gene expression microarrays (from Affymetrix), exon expression microarrays (from Affymetrix), and RNA-sequencing (from Illumina). Using these data and based on our previous studies to characterize the transcriptomic portrait of human mesenchymal stromal / stem cells; we perform a series of analysis in order to identify specific genes that are activated and expressed in a distinct and differential way in human BM-MSC. Moreover, we use a robust bioinformatic method, called GlobalTest, to assess the presence of an association between one or more genes expressed in a sample (measured as a set of ordinally scaled covariates) and an outcome variable as a single univariate response (i.e., an outcome that indicates the type of sample that is tested), done using a range of generalized linear models. In our case the outcome identifies the type of sample and, therefore, identifies the cell type of each sample tested based on the expression profile of specific gene or genes that are used as input in the test. Therefore, GlobalTest provides a robust statistical association method to elucidate whether sets of genes are significantly associated with a variable of interest, which in our case corresponds to the cell type. The method is based on a prediction model to detect a response variable using the expression measurements of the genes as input features. The null hypothesis tested is that the expression profile of a gene, within all the genes measured, is not associated with the response variable. The method also provides a statistical evaluation of the precision in the assignment and the strength of the association of a given feature to a specific phenotype, that is, to the response variable. Although the therapeutic potential of the MSC has been clearly established, there is still an unmet medical need of finding reliable strategies for identifying MSC in a cell population obtained from a subject and/or for differentiating MSC from FIB.

The present invention is thus focused on solving this problem and a reliable method for identifying MSC, and/or for differentiating MSC from FIB, is herein provided.

### DESCRIPTION OF THE INVENTION

### Brief description of the invention

As explained above, the present invention refers to an *in vitro* method for identifying MSC in a cell population obtained from a subject and/or for differentiating MSC from FIB. The present invention also refers to a substantially pure population of MSC, which have been isolated following the method described in the present invention.

The main objective of the present invention is to identify specific gene markers for MSC in comparison with other related cell types. In particular, the identification of gene markers that allow a clear distinction between MSC and FIB is of utmost relevance because, nowadays, the gold standard CD markers (i.e. gene ENG, that is CD105; gene THY1, that is CD90; and gene NT5E, that is CD73), that are used to identify and select human MSC are also expressed at similar levels in FIB. Therefore, MSC can often be erroneously identified as FIB, or vice versa.

Such as it is shown in the results provided below, the inventors of the present invention have demonstrated that the expression of the gene TAGLN (Transgelin) is an indication that the cells are MSC (and that they are not FIB). Moreover, the present invention also comprises assessing the expression of the gene COL4A1 (Collagen type IV alpha 1 chain) and/or COL4A2 (Collagen type IV alpha 2 chain), wherein the expression of the gene COL4A1 and/or COL4A2 is an indication that the cells are MSC and that the cells are not FIB.

Particularly, the results provided below show that TAGLN is the best gene marker of all the gene tested, presenting the best precision (value 1.0) and very good sensitivity=0.927 and specificity=1.0. TAGLN combined with COL4A1 (Collagen type IV alpha 1 chain) and/or COL4A2 (Collagen type IV alpha 2 chain) are also very good markers of MSC. In all cases these genes have less errors in the identification of MSC that the 3 standard genes (CD105, CD90 and CD73), that many times showed clear difficulties in the correct identification of FIB versus MSC.

So, taking into account these results, the first embodiment of the present invention refers to an *in vitro* method for identifying MSC in a cell population obtained from a subject, which comprises assessing the expression of the gene TAGLN in the cell population, wherein the expression of the gene TAGLN is an indication that the cells are MSC.

The second embodiment of the present invention refers to an *in vitro* method for differentiating MSC from FIB in a cell population obtained from a subject, which comprises assessing the expression of the gene TAGLN, wherein the expression of the gene TAGLN is an indication that the cells are MSC and that the cells are not FIB.

In a preferred embodiment, the method of the invention further comprises assessing the expression of the gene COL4A1 and/or COL4A2, wherein the expression of the gene COL4A1 and/or COL4A2 is an indication that the cells are MSC and that the cells are not FIB.

In a preferred embodiment, the method of the invention comprises measuring the expression levels of the genes TAGLN, or [TAGLN and COL4A1], or [TAGLN and COL4A2], wherein if an overexpression of the genes is identified with respect to a pre-established threshold value determined in cells which are not MSC, it is an indication that the cells are MSC and that the cells are not FIB.

The third embodiment of the present invention refers to the *in vitro* use of TAGLN for identifying MSC, or for differentiating MSC from FIB, in a cell population obtained from a subj ect.

In a preferred embodiment, the present invention refers to the *in vitro* use of [TAGLN and COL4A1], or [TAGLN and COL4A2], for identifying MSC, or for differentiating MSC from FIB, in a cell population obtained from a subject.

In a preferred embodiment, the present invention refers to the *in vitro* use of a kit comprising reagents for measuring the expression of TAGLN for identifying MSC, or for differentiating MSC from FIB, in a cell population obtained from a subject.

In a preferred embodiment, the present invention refers to the *in vitro* use of a kit comprising reagents for measuring the expression of [TAGLN and COL4A1], or [TAGLN and COL4A2], for identifying MSC, or for differentiating MSC from FIB, in a cell population obtained from a subject.

The fourth embodiment of the present invention refers to a kit consisting of reagents for measuring the expression of [TAGLN and COL4A1] or [TAGLN and COL4A2].

The fifth embodiment of the present invention refers to a substantially pure population of MSC (hereinafter the "substantially pure population of MSC of the invention"), wherein the MSC are at least 80% of the total cell population, characterized by the expression of the gene TAGLN.

In a preferred embodiment, the present invention refers to a substantially pure population of MSC characterized by the expression of the genes [TAGLN and COL4A1] or [TAGLN and COL4A2].

In a preferred embodiment, the present invention refers to a substantially pure population of MSC characterized by overexpressing of the genes TAGLN, or [TAGLN and COL4A1], or [TAGLN and COL4A2], with respect to a pre-established threshold value determined in cells which are not MSC.

The sixth embodiment of the present invention refers to a pharmaceutical composition comprising the above defined substantially pure population of MSC of the invention and, optionally, pharmaceutically acceptable excipients or carriers.

The seventh embodiment of the present invention refers to a substantially pure population of MSC of the invention, or to a pharmaceutical composition comprising the substantially pure population of MSC of the invention, for use as a medicament.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.
- "Pharmaceutically acceptable excipient or carrier" refers to an excipient that may optionally be included in the compositions of the invention and that causes no significant adverse toxicological effects to the patient.

### Description of the figures

**Figure 1****.** Global Tests done on 3 datasets with 3 different cell-types tested using the MSC standard genes: ENG(CD105), THY1(CD90), NT5E(CD73). **A)** Dataset 1 (gene expression microarrays): 70 samples (11 FIB, 10 HSC, 49 MSC) = 18 mistaken^{∗}. **B**) Dataset 2 (exon expression microarrays): 15 samples (3 FIB, 3 HSC, 9 MSC) = 3 mistaken^{∗}. **C)** Dataset 3 (pair-end RNA-sequencing): 29 samples (8 FIB, 15 HSC, 6 MSC) = 0 mistaken^{∗}. Y axis (weighted posterior effect correlation).
**Figure 2**. Results of the Global Tests including the confusion matrices to show the statistical measurement of the error in the assignment to MSC cell type of samples from 3 independent datasets (114 samples produced with 3 different genome-wide expression platforms), testing 7 single genes.
**Figure 3**. Results of the Global Tests including the confusion matrices to show the statistical measurement of the error in the assignment to MSC cell type of samples from 3 independent datasets (114 samples produced with 3 different expression platforms), testing combinations of 2, 3 or 4 genes.
**Figure 4**. Results of the RT-PCR done to quantify the expression of 9 genes in 6 human cell-types.

### Detailed description of the invention

The present invention is illustrated by means of the examples set below, without the intention of limiting its scope of protection.

### Example 1. Material and Methods

### Example 1.1. Human cell samples to test the markers

For the current study, samples from different human cell types were obtained from different sources, as described in **Table 1.**

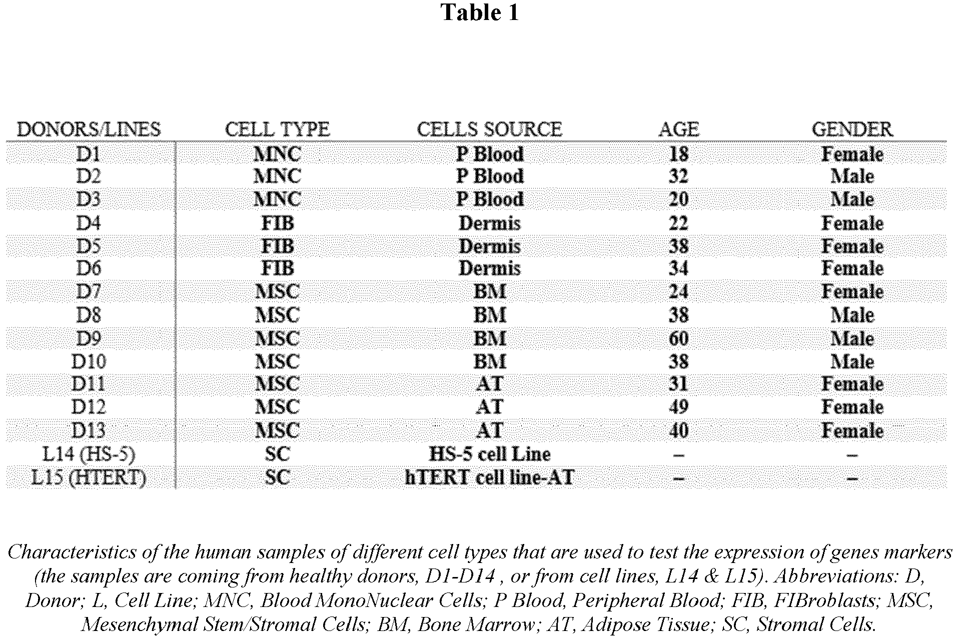

In this way we obtained Mononuclear Cells (MNC) from peripheral blood (n=3 samples); Mesenchymal Stem/Stromal Cells isolated from bone marrow (BM-MSC, n=4 samples); and Mesenchymal Stem/Stromal Cells isolated from adipose tissue (AT-MSC, n=3 samples). All these were obtained from healthy donors. Furthermore, human primary Fibroblasts (FIB, n=3) from dermis (from 3 different female donors), were purchased from *Innoprot* (https://innoprot.com/); and two stromal cell lines (SC, n=2) cited below. All samples from donors obtained in our Hospital (MNCs, BM-MSC at AT-MSC) where drawn after the corresponding informed consent was obtained in accordance with the Ethical Standards and Good Clinical Practice established by the Ethics Committee of the University Hospital of Salamanca.

### Example 1.2. Cell processing

Mononuclear Cells (MNC) isolated from peripheral blood: 5ml of peripheral blood was collected in EDTA tubes from 3 healthy donors. Peripheral blood was lysed two times with 1X ammonium chloride for 20min and 5min respectively in cold, after incubation washed twice with PBS. The pellet was mixed with trizol and RNA was extracted (see section 2). Fibroblasts (FIB): Cryopreserved Fibroblast cells from 3 different adults (purchased from *Innoprot*) were thawed and seeded at the rate of 5000 cells/cm² in a culture flask with Fibroblast medium supplemented with 2% Fetal bovine Serum (FBS), 1% of Fibroblast Growth Supplement (FGS) and 1% of penicillin/streptomycin solution until confluence was reached. In this moment, cells were trypsinized and subcultured to obtain the needed number for further experiments. Bone marrow collection: Bone marrow extraction was performed under locoregional anesthesia, by puncture in the postero-superior iliac spine. Mononuclear cells from the bone marrow were obtained by density gradient by Ficoll-PaqueTM Plus (density: 1.077k, GE Healthcare Bio-Sciences AB). They were centrifuged for 30 minutes at 500g. Finally, the mononuclear cells recovered from the interphase were washed 2 times with HBSS medium (Hank's Balanced Salt Solution with Phenol Red, BioWhittaker Lonza Verviers, Belgium) for 10 minutes at 300g.

Adipose tissue procurement: Human adipose tissue was obtained from liposuction under general anesthesia. Briefly, we disintegrate 1gr of adipose tissue from the liposuction and incubate it with collagenase at 37°C for one hour in agitation. Then, we pass the homogenized material through a 40µm filter and centrifuge the obtained filtrate. After lysing the cell button with ACK 1X lysing Buffer (A10492 Gibco, Invitrogen) mononuclear cells were washed two times with PBS.

Isolation and expansion of MSC from BM and AT: The mononuclear cells obtained from BM and the cells from AT were seeded at a rate of 1×10⁶/cm² in Dulbecco's Modified Eagle's medium-low glucose (DMEN,Gibco Invitrogen Paisely,UK) supplemented with 10% Bovine Serum Fetal (SBF, BioWhittaker^{®} Lonza, Verviers, Belgium) and 1% Penicillin/Estreptomycin (Gibco, Invitrogen, Paisely, UK). Every 2-3 days, the culture medium was changed until 80-90% confluence was reached. At this time, all culture medium was removed from the flask, washed with sterile PBS (GIBCO Invitrogen Corporation, Paisely, UK) and incubated with 0.05% 1X trypsin (GIBCO Invitrogen Corporation, Paisely, UK) for 5 minutes at 37°C. Cells were subsequently seeded at a concentration of 5,000 cells/cm² in culture flasks with a larger surface area, until reaching passage 3, which was used for this study.

Stromal cell lines: We used two human stromal immortalized cell lines (hTERT and HS-5), both grown with medium expansion of mesenchymal (DMEN, 10%FBS and 1% P/S). The former is a human MSC line immortalized by expression of the telomerase reverse transcriptase gene (hMSC-TERT) was a generous gift from Dr. D Campana (Professor, Department of Paediatrics, Yong Loo Lin School of Medicine, National University of Singapore); whereas cell line HS-5 is derived from bone marrow stroma (HS-5, ATCC^{®} CRL-11882^{™}, https://www.atcc.org/products/crl-11882).

### Example 1.3. Quantitative gene expression measurements

All cells were collected, and RNA was extracted by the thiocyanate-phenol-chloroform method. To measure the concentration and the RNA integrity, the Agilent 2100 Bioanalyzer system (Agilent, Palo Alto, CA) was used. Reverse transcription was performed, using the High Capacity kit (Applied Biosystems, Foster City, CA, USA). For quantitative PCR, the Step One Plus Real-Time PCR System (Applied Biosystems, Foster City, CA) was used with commercial TaqMan^{®} Gene Expression Assays (Applied Biosystems, Foster City, CA). The genes analyzed are shown in **Table 2,** as well as the identification number of each gene tested.

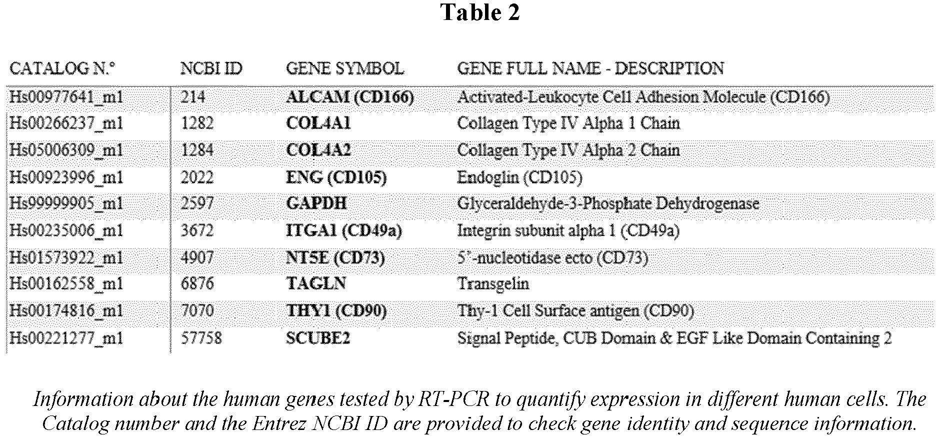

To quantify the expression of each query gene with respect to the control gene (GADPH), we calculated the formula 2-ΔCt where: ΔCt = Ct study gene - Ct GADPH. Differences between the expression signals obtained with the different cell types were analyzed to identify the significant differences (i.e. p-values <0.05) using a non-parametric Mann-Whitney U test between samples.

### Example 1.4. Datasets compiled and used to test the gene markers

Three independent datasets including genome-wide expression data from three different human primary cells (Hematopoietic Stem Cells, HSC; Mesenchymal Stem/Stromal Cells, MSC; and primary Fibroblasts, FIB) were collected, normalized from raw data and quantified to produce full gene expression data. The datasets were produced using three different transcriptomic platforms: **(i)** gene expression *Affymetrix* high-density microarrays; **(ii)** exon expression *Affymetrix* high-density microarrays; and **(iii)** *Illumina* pair-end RNA-sequencing technique. The *first* dataset included 70 samples corresponding to gene expression microarrays that were obtained as a selection from the 264 samples and 18 datasets included in a large metanalysis from public data bases. Therefore, this first dataset included 49 MSC samples, isolated from bone marrow; 10 samples of hematopoietic stem cells, HSC; and 11 samples of skin-derived primary FIB. The *second* dataset included 15 samples of exon expression arrays produced in our lab, that includes 3 samples of adipose derived MSC, AT-MSC; 3 MSC from bone marrow, BM-MSC; 3 MSC samples from placenta, PL-MSC; 3 HSC; and 3 samples of dermal Fibroblasts, FIB; all tested using the Human Exon 1.0 array platform (*Affymetrix*)*,* which is fully accessible from GEO database under the identifier GSE72332. Finally, the *third* dataset corresponds to a compendium subset from 8 expression datasets, all produced with *Illumina* RNA-sequencing technology (platforms *Illumina* HiSeq 2000, 2500 or 4000), from which we selected the raw files and normalized together 29 samples of our interest: 6 samples from bone marrow mesenchymal stem cells, BM-MSC; 15 samples from bone marrow HSCs; and 8 are from skin Fibroblasts, FIB. The original datasets that include these samples are accessible in GEO with the following identifiers: GSE51518, GSE63569, GSE74053, GSE81478, GSE102881, GSE105145, GSE114922, GSE119501.

### Example 1.5. Statistical computational data analyses for cell-specific gene marker identification

Using R (version 4.0.4, https://cran.r-project.org/) computational language for statistical programming and different software packages from Bioconductor (https://www.bioconductor.org/), we implemented a series of bioinformatic computational protocols and methods to analyse the gene expression signatures of samples from different cell types that are closely related (i.e., MSC, HSC and FIB), with the objective of identifying one or more genes that were specific and selective markers of the MSC isolated from human bone marrow: BM-MSC. As indicated above we have 3 independent gene expression datasets with 70, 15 and 29 samples produced by three different transcriptomic platforms. Using this data we applied a robust algorithm, called *Global Test,* that allows testing groups of features for significant association with a response variable (that is, test all features, that in our case are the genes, as candidate markers associated with a given subset of samples, that in our case are the different cell types). The algorithm is based on multiple regression to adjust the model by selecting the best features in the role of proposed markers, which influence a response variable because show significant association or correlation with the different sample classes. The results provide a statistical test and p-value to weigh such an association between the selected features (i.e., the selected genes) and the output status (i.e. the sample subtype or cell type).

### Example 2. Results

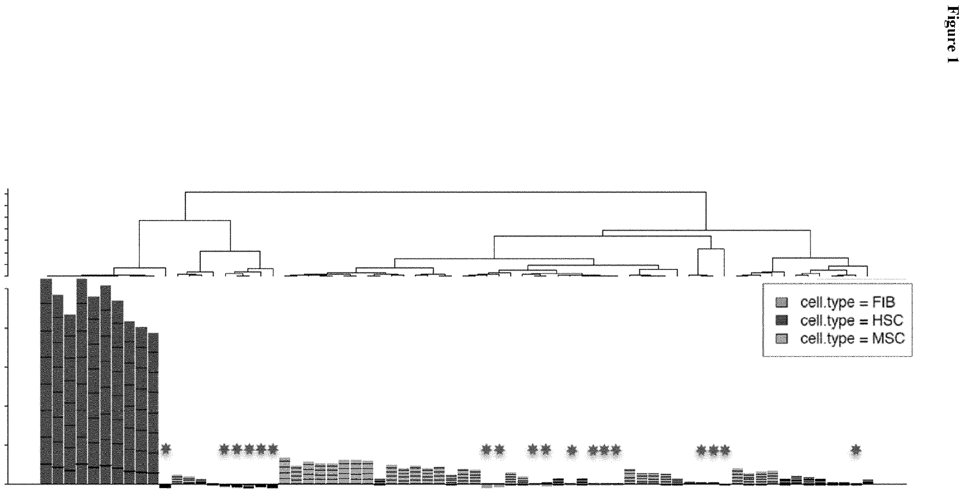

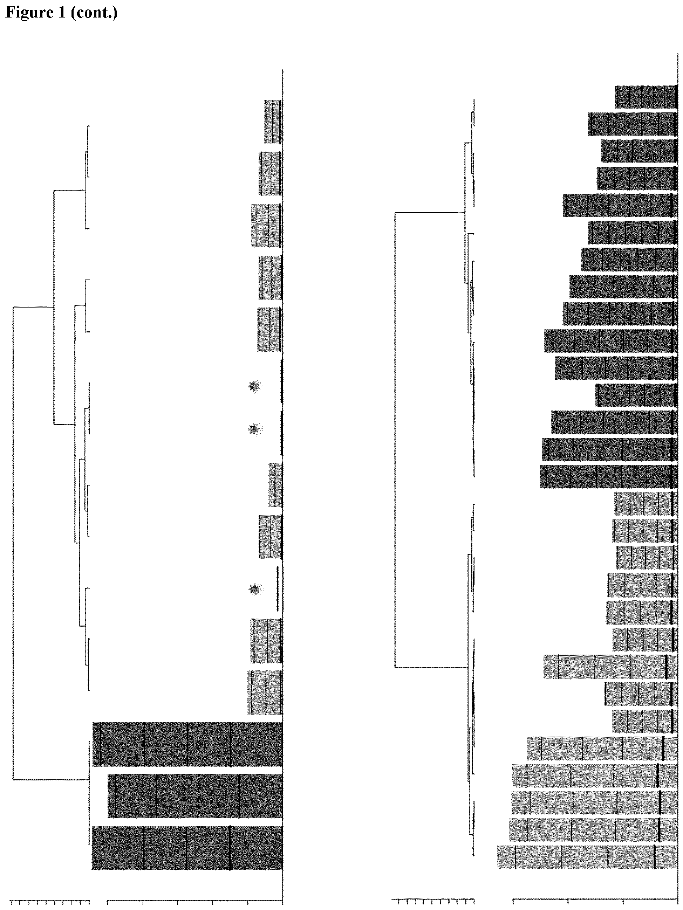

A deep analyses of multiple genome-wide expression data has been done in the present invention to find novel genes that present a more distinctive expression in MSC versus other types of cells. After the analysis of multiple transcriptomic profiles, we found 4 candidate genes that we tested in a very specific way in this work. These candidate genes are: TAGLN, COL4A1, COL4A2 and SCUBE3 (all described and referenced in **Table 2)**. To perform a deep and robust testing of these genes as expression markers of MSC, we collected and prepared 3 independent gene expression datasets with 70, 15 and 29 samples produced by three different transcriptomic platforms, that include three different human primary cells: MSC, HSC and FIB. Using these datasets, we applied *Global Test,* that is a robust predictive algorithm to identify if one or more genes (i.e., one or more measured biomolecular features or characteristics) are accurate markers of a given class of samples. Note that in our case the "classes" of the samples are the "cell types" of the samples, since we are looking for specific markers for specific cell types. Using this method, we tested 3 datasets using as candidate markers gold standard genes described above: ENG (CD105), THY1 (CD90) and NT5E (CD73). The results of this analysis are presented in **Figure 1** that shows the output of the *Global Test,* revealing how many of the 114 samples are well identified and classified in the correct cell type. In the case of dataset 1, **Figure 1A,** we found 18 samples mistaken (not well classified); and in the case of dataset 2, **Figure 1B,** we found 3 samples mistaken (not well classified). The same analysis was performed with this compendium of 114 samples for each of the individual genes tested and combinations of genes tested, producing a total 11 different analyses where sample assignment and error rates were calculated. All the detailed parameters and results of these analyses are included in below.

### TAGLN

| **DT1: Microarrays (Metanalysis) (N=70)** | | | | | |
|---|---|---|---|---|---|
| **Cell-types** | **p-value** | **Statistic** | **Expected** | **Std.dev** | **Residual** |
| **HSC** | 5.67E-15 | 45.92700 | 2.91268 | 5.56949 | 2 |
| **HSC** | 5.91E-15 | 48.21294 | 3.20985 | 5.83088 | 2 |
| **HSC** | 5.97E-15 | 48.94146 | 3.30758 | 5.91370 | 2 |
| **HSC** | 6.30E-15 | 53.94719 | 4.01878 | 6.47597 | 2 |
| **HSC** | 6.33E-15 | 54.85446 | 4.15509 | 6.57655 | 2 |
| **HSC** | 6.35E-15 | 55.28518 | 4.22060 | 6.62415 | 2 |
| **HSC** | 6.35E-15 | 55.47236 | 4.24923 | 6.64480 | 2 |
| **HSC** | 6.36E-15 | 55.73389 | 4.28939 | 6.67363 | 2 |
| **HSC** | 6.37E-15 | 56.13990 | 4.35211 | 6.71831 | 2 |
| **HSC** | 6.38E-15 | 58.08401 | 4.65876 | 6.93104 | 2 |
| **MSC** | 1.05E-12 | 0.74258 | 0.00050 | 0.10559 | 3 |
| **MSC** | 1.14E-12 | 3.13550 | 0.00894 | 0.44564 | 3 |
| **MSC** | 1.18E-12 | 4.40322 | 0.01763 | 0.62552 | 3 |
| **MSC** | 1.19E-12 | 4.55630 | 0.01887 | 0.64723 | 3 |
| **MSC** | 1.19E-12 | 4.61564 | 0.01937 | 0.65564 | 3 |
| **MSC** | 1.19E-12 | 4.80653 | 0.02100 | 0.68270 | 3 |
| **MSC** | 1.21E-12 | 5.58542 | 0.02836 | 0.79301 | 3 |
| **MSC** | 1.21E-12 | 5.63587 | 0.02888 | 0.80015 | 3 |
| **MSC** | 1.22E-12 | 5.76893 | 0.03026 | 0.81899 | 3 |
| **MSC** | 1.22E-12 | 5.82579 | 0.03086 | 0.82703 | 3 |
| **MSC** | 1.22E-12 | 5.87212 | 0.03135 | 0.83359 | 3 |
| **MSC** | 1.22E-12 | 6.11866 | 0.03404 | 0.86846 | 3 |
| **MSC** | 1.23E-12 | 6.22722 | 0.03525 | 0.88381 | 3 |
| **MSC** | 1.23E-12 | 6.23768 | 0.03537 | 0.88529 | 3 |
| **MSC** | 1.23E-12 | 6.26764 | 0.03571 | 0.88953 | 3 |
| **MSC** | 1.23E-12 | 6.27431 | 0.03579 | 0.89047 | 3 |
| **MSC** | 1.23E-12 | 6.27521 | 0.03580 | 0.89060 | 3 |
| **MSC** | 1.23E-12 | 6.30956 | 0.03619 | 0.89545 | 3 |
| **MSC** | 1.23E-12 | 6.67497 | 0.04051 | 0.94709 | 3 |
| **MSC** | 1.23E-12 | 6.71593 | 0.04100 | 0.95288 | 3 |
| **MSC** | 1.24E-12 | 6.73109 | 0.04119 | 0.95502 | 3 |
| **MSC** | 1.24E-12 | 6.74046 | 0.04130 | 0.95634 | 3 |
| **MSC** | 1.24E-12 | 6.75205 | 0.04145 | 0.95798 | 3 |
| **MSC** | 1.24E-12 | 6.82587 | 0.04236 | 0.96841 | 3 |
| **MSC** | 1.24E-12 | 6.89049 | 0.04316 | 0.97753 | 3 |
| **MSC** | 1.24E-12 | 6.89286 | 0.04319 | 0.97787 | 3 |
| **MSC** | 1.24E-12 | 7.10616 | 0.04591 | 1.00798 | 3 |
| **MSC** | 1.24E-12 | 7.16383 | 0.04666 | 1.01612 | 3 |
| **MSC** | 1.24E-12 | 7.19548 | 0.04707 | 1.02059 | 3 |
| **MSC** | 1.24E-12 | 7.23112 | 0.04754 | 1.02562 | 3 |
| **MSC** | 1.24E-12 | 7.31725 | 0.04868 | 1.03777 | 3 |
| **MSC** | 1.24E-12 | 7.38699 | 0.04961 | 1.04761 | 3 |
| **MSC** | 1.25E-12 | 7.53238 | 0.05158 | 1.06811 | 3 |
| **MSC** | 1.25E-12 | 7.79706 | 0.05527 | 1.10543 | 3 |
| **MSC** | 1.25E-12 | 7.81583 | 0.05554 | 1.10807 | 3 |
| **MSC** | 1.25E-12 | 7.86234 | 0.05620 | 1.11463 | 3 |
| **MSC** | 1.25E-12 | 7.93897 | 0.05730 | 1.12543 | 3 |
| **MSC** | 1.25E-12 | 7.99291 | 0.05808 | 1.13302 | 3 |
| **MSC** | 1.25E-12 | 8.01147 | 0.05835 | 1.13564 | 3 |
| **MSC** | 1.25E-12 | 8.22455 | 0.06150 | 1.16565 | 3 |
| **MSC** | 1.25E-12 | 8.29514 | 0.06256 | 1.17559 | 3 |
| **MSC** | 1.25E-12 | 8.29940 | 0.06262 | 1.17618 | 3 |
| **MSC** | 1.25E-12 | 8.43936 | 0.06475 | 1.19588 | 3 |
| **MSC** | 1.25E-12 | 8.44869 | 0.06489 | 1.19720 | 3 |
| **MSC** | 1.25E-12 | 8.56133 | 0.06664 | 1.21304 | 3 |
| **MSC** | 1.26E-12 | 8.70524 | 0.06889 | 1.23329 | 3 |
| **MSC** | 1.26E-12 | 8.75668 | 0.06971 | 1.24052 | 3 |
| **MSC** | 1.26E-12 | 8.85589 | 0.07130 | 1.25447 | 3 |
| **MSC** | 1.26E-12 | 9.52072 | 0.08241 | 1.34784 | 3 |
| **FIB** | 8.58E-06 | 3.37720 | 0.09644 | 0.76316 | 1 |
| **FIB** | 8.83E-06 | 3.56805 | 0.10764 | 0.80612 | 1 |
| **FIB** | 1.00E-05 | 4.46178 | 0.16832 | 1.00696 | 1 |
| **FIB** | 1.01E-05 | 4.49063 | 0.17051 | 1.01343 | 1 |
| **FIB** | 1.79E-05 | 8.93386 | 0.67484 | 1.99800 | 1 |
| **FIB** | 1.89E-05 | 9.43056 | 0.75197 | 2.10614 | 1 |
| **FIB** | 2.06E-05 | 10.19533 | 0.87888 | 2.27168 | 1 |
| **FIB** | 0.9999951 | -0.10965 | 0.00010 | 0.02482 | 1 |
| **FIB** | 0.9999962 | -1.51975 | 0.01953 | 0.34389 | 1 |
| **FIB** | 0.9999963 | -1.64647 | 0.02292 | 0.37254 | 1 |
| **FIB** | 0.9999965 | -1.92546 | 0.03135 | 0.43560 | 1 |

| | **DT2: Exon Arrays (N=15)** | | | | |
|---|---|---|---|---|---|
| **Cell-types** | **p-value** | **Statistic** | **Expected** | **Std.dev** | **Residual** |
| **MSC** | 0.000323 | 2.23090 | 0.05855 | 0.63675 | 3 |
| **MSC** | 0.000339 | 3.00924 | 0.10652 | 0.85414 | 3 |
| **MSC** | 0.000342 | 3.19453 | 0.12005 | 0.90530 | 3 |
| **MSC** | 0.000347 | 5.11562 | 0.30785 | 1.41745 | 3 |
| **MSC** | 0.000348 | 3.80588 | 0.17039 | 1.07217 | 3 |
| **MSC** | 0.000349 | 3.88408 | 0.17747 | 1.09328 | 3 |
| **MSC** | 0.000349 | 3.95920 | 0.18440 | 1.11350 | 3 |
| **MSC** | 0.00035 | 4.70216 | 0.26009 | 1.31049 | 3 |
| **MSC** | 0.00035 | 4.10191 | 0.19793 | 1.15178 | 3 |
| **HSC** | 0.000577 | 10.61920 | 2.20532 | 2.58892 | 2 |
| **HSC** | 0.000609 | 12.08816 | 2.85764 | 2.85375 | 2 |
| **HSC** | 0.000612 | 12.54261 | 3.07654 | 2.92783 | 2 |
| **FIB** | 0.032908 | 0.32115 | 0.01508 | 0.16637 | 1 |
| **FIB** | 0.040266 | 0.67235 | 0.06610 | 0.34690 | 1 |
| **FIB** | 0.060582 | 1.48239 | 0.32132 | 0.74912 | 1 |

| | **DT3: RNA-seq (N=29)** | | | | |
|---|---|---|---|---|---|
| **Cell-types** | **p-value** | **Statistic** | **Expected** | **Std.dev** | **Residual** |
| **HSC** | 5.05E-07 | 2.60901 | 0.02557 | 0.52836 | 2 |
| **HSC** | 6.06E-07 | 6.02290 | 0.13627 | 1.21282 | 2 |
| **HSC** | 6.11E-07 | 13.32976 | 0.66750 | 2.60962 | 2 |
| **HSC** | 6.17E-07 | 6.57208 | 0.16226 | 1.32163 | 2 |
| **HSC** | 6.26E-07 | 12.54552 | 0.59126 | 2.46628 | 2 |
| **HSC** | 6.28E-07 | 7.17068 | 0.19316 | 1.43970 | 2 |
| **HSC** | 6.36E-07 | 7.70614 | 0.22309 | 1.54480 | 2 |
| **HSC** | 6.40E-07 | 8.01750 | 0.24148 | 1.60568 | 2 |
| **HSC** | 6.42E-07 | 11.40493 | 0.48864 | 2.25447 | 2 |
| **HSC** | 6.46E-07 | 8.61383 | 0.27874 | 1.72176 | 2 |
| **HSC** | 6.47E-07 | 8.73096 | 0.28637 | 1.74447 | 2 |
| **HSC** | 6.48E-07 | 8.89365 | 0.29714 | 1.77597 | 2 |
| **HSC** | 6.50E-07 | 9.49380 | 0.33860 | 1.89169 | 2 |
| **HSC** | 6.50E-07 | 10.05303 | 0.37966 | 1.99879 | 2 |
| **HSC** | 6.50E-07 | 9.62314 | 0.34789 | 1.91653 | 2 |
| **MSC** | 1.73E-05 | 19.89851 | 1.74966 | 4.38216 | 3 |
| **MSC** | 2.03E-05 | 23.63456 | 2.46835 | 5.15677 | 3 |
| **MSC** | 2.09E-05 | 24.44469 | 2.64047 | 5.32153 | 3 |
| **MSC** | 2.31E-05 | 27.33833 | 3.30260 | 5.89956 | 3 |
| **MSC** | 2.32E-05 | 27.44580 | 3.32862 | 5.92070 | 3 |
| **MSC** | 2.34E-05 | 27.70997 | 3.39300 | 5.97254 | 3 |
| **FIB** | 0.0008701 | 0.90825 | 0.01676 | 0.28470 | 1 |
| **FIB** | 0.0010186 | 1.71455 | 0.05974 | 0.53645 | 1 |
| **FIB** | 0.0010821 | 2.04323 | 0.08484 | 0.63859 | 1 |
| **FIB** | 0.0011974 | 2.62119 | 0.13963 | 0.81729 | 1 |
| **FIB** | 0.0013281 | 3.25385 | 0.21517 | 1.01122 | 1 |
| **FIB** | 0.0014062 | 3.62365 | 0.26685 | 1.12360 | 1 |
| **FIB** | 0.0016002 | 4.52573 | 0.41625 | 1.39409 | 1 |
| **FIB** | 0.0017062 | 5.01455 | 0.51103 | 1.53814 | 1 |

| **COL4A1** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **MSC** | 1.30E-14 | 0.29251 | 0.00006 | 0.03839 | 3 |
| **MSC** | 1.33E-14 | 1.07176 | 0.00078 | 0.14067 | 3 |
| **MSC** | 1.38E-14 | 2.15480 | 0.00315 | 0.28279 | 3 |
| **MSC** | 1.40E-14 | 2.45474 | 0.00408 | 0.32213 | 3 |
| **MSC** | 1.40E-14 | 2.58174 | 0.00452 | 0.33879 | 3 |
| **MSC** | 1.43E-14 | 3.32376 | 0.00748 | 0.43611 | 3 |
| **MSC** | 1.44E-14 | 3.42841 | 0.00796 | 0.44983 | 3 |
| **MSC** | 1.44E-14 | 3.57903 | 0.00868 | 0.46957 | 3 |
| **MSC** | 1.46E-14 | 4.03070 | 0.01101 | 0.52878 | 3 |
| **MSC** | 1.47E-14 | 4.35156 | 0.01283 | 0.57082 | 3 |
| **MSC** | 1.47E-14 | 4.40281 | 0.01313 | 0.57754 | 3 |
| **MSC** | 1.50E-14 | 5.24408 | 0.01863 | 0.68771 | 3 |
| **MSC** | 1.51E-14 | 15.96790 | 0.17272 | 2.07894 | 3 |
| **MSC** | 1.51E-14 | 5.57931 | 0.02109 | 0.73159 | 3 |
| **MSC** | 1.53E-14 | 15.13516 | 0.15517 | 1.97216 | 3 |
| **MSC** | 1.54E-14 | 14.78178 | 0.14801 | 1.92676 | 3 |
| **MSC** | 1.54E-14 | 14.54595 | 0.14332 | 1.89644 | 3 |
| **MSC** | 1.54E-14 | 14.51927 | 0.14280 | 1.89301 | 3 |
| **MSC** | 1.55E-14 | 14.42578 | 0.14097 | 1.88098 | 3 |
| **MSC** | 1.55E-14 | 14.32308 | 0.13897 | 1.86777 | 3 |
| **MSC** | 1.55E-14 | 14.24673 | 0.13749 | 1.85794 | 3 |
| **MSC** | 1.55E-14 | 14.21436 | 0.13686 | 1.85378 | 3 |
| **MSC** | 1.56E-14 | 13.83755 | 0.12970 | 1.80524 | 3 |
| **MSC** | 1.56E-14 | 13.71096 | 0.12734 | 1.78893 | 3 |
| **MSC** | 1.56E-14 | 13.54619 | 0.12430 | 1.76768 | 3 |
| **MSC** | 1.57E-14 | 13.28990 | 0.11964 | 1.73462 | 3 |
| **MSC** | 1.57E-14 | 13.24564 | 0.11885 | 1.72891 | 3 |
| **MSC** | 1.57E-14 | 8.30921 | 0.04677 | 1.08825 | 3 |
| **MSC** | 1.58E-14 | 12.70621 | 0.10936 | 1.65924 | 3 |
| **MSC** | 1.58E-14 | 12.57384 | 0.10710 | 1.64213 | 3 |
| **MSC** | 1.58E-14 | 12.21426 | 0.10106 | 1.59562 | 3 |
| **MSC** | 1.58E-14 | 12.14464 | 0.09991 | 1.58661 | 3 |
| **MSC** | 1.58E-14 | 9.35457 | 0.05928 | 1.22444 | 3 |
| **MSC** | 1.58E-14 | 11.98611 | 0.09732 | 1.56609 | 3 |
| **MSC** | 1.58E-14 | 11.62855 | 0.09160 | 1.51978 | 3 |
| **MSC** | 1.59E-14 | 9.92207 | 0.06669 | 1.29827 | 3 |
| **MSC** | 1.59E-14 | 10.01014 | 0.06788 | 1.30972 | 3 |
| **MSC** | 1.59E-14 | 11.42378 | 0.08840 | 1.49324 | 3 |
| **MSC** | 1.59E-14 | 11.35543 | 0.08735 | 1.48438 | 3 |
| **MSC** | 1.59E-14 | 11.28405 | 0.08625 | 1.47512 | 3 |
| **MSC** | 1.59E-14 | 11.14145 | 0.08409 | 1.45663 | 3 |
| **MSC** | 1.59E-14 | 11.10387 | 0.08352 | 1.45176 | 3 |
| **MSC** | 1.59E-14 | 10.44391 | 0.07389 | 1.36609 | 3 |
| **MSC** | 1.59E-14 | 10.98523 | 0.08174 | 1.43636 | 3 |
| **MSC** | 1.59E-14 | 10.82591 | 0.07939 | 1.41569 | 3 |
| **MSC** | 1.59E-14 | 10.80865 | 0.07914 | 1.41345 | 3 |
| **HSC** | 1.43E-12 | 34.67921 | 1.77808 | 4.71048 | 2 |
| **HSC** | 1.54E-12 | 36.72727 | 1.99429 | 4.98029 | 2 |
| **HSC** | 1.69E-12 | 39.44791 | 2.30070 | 5.33644 | 2 |
| **HSC** | 1.91E-12 | 43.46273 | 2.79284 | 5.85688 | 2 |
| **HSC** | 1.97E-12 | 44.59704 | 2.94052 | 6.00274 | 2 |
| **HSC** | 2.03E-12 | 45.75436 | 3.09511 | 6.15099 | 2 |
| **HSC** | 2.07E-12 | 46.62182 | 3.21359 | 6.26172 | 2 |
| **HSC** | 2.09E-12 | 47.03904 | 3.27136 | 6.31486 | 2 |
| **HSC** | 2.10E-12 | 47.29060 | 3.30645 | 6.34686 | 2 |
| **HSC** | 2.13E-12 | 47.83452 | 3.38294 | 6.41596 | 2 |
| **FIB** | 1.24E-09 | 4.52955 | 0.08055 | 0.74619 | 1 |
| **FIB** | 1.36E-09 | 5.27151 | 0.10910 | 0.86803 | 1 |
| **FIB** | 1.59E-09 | 6.58416 | 0.17019 | 1.08315 | 1 |
| **FIB** | 1.64E-09 | 6.80247 | 0.18167 | 1.11887 | 1 |
| **FIB** | 1.66E-09 | 6.95204 | 0.18975 | 1.14332 | 1 |
| **FIB** | 3.20E-09 | 13.50423 | 0.71595 | 2.20265 | 1 |
| **FIB** | 3.74E-09 | 15.39543 | 0.93053 | 2.50259 | 1 |
| **FIB** | 3.91E-09 | 15.98002 | 1.00254 | 2.59464 | 1 |
| **FIB** | 3.98E-09 | 16.24682 | 1.03629 | 2.63654 | 1 |
| **FIB** | 5.33E-09 | 20.78150 | 1.69551 | 3.33674 | 1 |
| **FIB** | 5.77E-09 | 22.36070 | 1.96298 | 3.57461 | 1 |
| **MSC** | 1.00E+00 | -2.11059 | 0.00302 | 0.27699 | 3 |
| **MSC** | 1.00E+00 | -1.03309 | 0.00072 | 0.13559 | 3 |
| **MSC** | 1.00E+00 | -5.01473 | 0.01703 | 0.65768 | 3 |

| | **DT2: Exon Arrays (N=15)** | | | | |
|---|---|---|---|---|---|
| **Cell-types** | **p-value** | **Statistic** | **Expected** | **Std.dev** | **Residual** |
| **MSC** | 0.0003292 | 2.48350 | 0.06145 | 0.71103 | 3 |
| **MSC** | 0.0003304 | 7.72540 | 0.59464 | 2.09399 | 3 |
| **MSC** | 0.0003310 | 7.70167 | 0.59099 | 2.08839 | 3 |
| **MSC** | 0.0003402 | 7.28566 | 0.52887 | 1.98884 | 3 |
| **MSC** | 0.0003519 | 3.77221 | 0.14178 | 1.07153 | 3 |
| **MSC** | 0.0003556 | 4.09899 | 0.16740 | 1.16140 | 3 |
| **MSC** | 0.0003583 | 4.40753 | 0.19355 | 1.24558 | 3 |
| **MSC** | 0.0003594 | 5.82218 | 0.33774 | 1.62151 | 3 |
| **HSC** | 0.0038321 | 7.77332 | 1.43223 | 2.37804 | 2 |
| **HSC** | 0.0041068 | 8.35470 | 1.65447 | 2.53493 | 2 |
| **HSC** | 0.0048964 | 10.05250 | 2.39523 | 2.96442 | 2 |
| **FIB** | 0.0125174 | 1.81460 | 0.23505 | 0.70489 | 1 |
| **FIB** | 0.0153068 | 2.38145 | 0.40483 | 0.91423 | 1 |
| **FIB** | 0.0195987 | 3.16279 | 0.71406 | 1.18749 | 1 |
| **MSC** | 0.9998502 | -3.15038 | 0.09889 | 0.89868 | 3 |

| | **DT3: RNA-seq (N=29)** | | | | |
|---|---|---|---|---|---|
| **Cell-types** | **p-value** | **Statistic** | **Expected** | **Std.dev** | **Residual** |
| **HSC** | 4.61E-06 | 0.03663 | 0.00001 | 0.00826 | 2 |
| **HSC** | 5.07E-06 | 1.21694 | 0.00628 | 0.27428 | 2 |
| **HSC** | 5.23E-06 | 19.21799 | 1.56703 | 4.00487 | 2 |
| **HSC** | 5.23E-06 | 19.21799 | 1.56703 | 4.00487 | 2 |
| **HSC** | 5.30E-06 | 1.78588 | 0.01353 | 0.40237 | 2 |
| **HSC** | 6.35E-06 | 4.78239 | 0.09704 | 1.07332 | 2 |
| **HSC** | 6.70E-06 | 6.02435 | 0.15399 | 1.34844 | 2 |
| **HSC** | 6.86E-06 | 6.66668 | 0.18857 | 1.48978 | 2 |
| **HSC** | 6.86E-06 | 6.69174 | 0.18999 | 1.49528 | 2 |
| **HSC** | 7.11E-06 | 7.91822 | 0.26602 | 1.76296 | 2 |
| **HSC** | 7.22E-06 | 12.80797 | 0.69602 | 2.79263 | 2 |
| **HSC** | 7.26E-06 | 9.02449 | 0.34555 | 2.00164 | 2 |
| **HSC** | 7.33E-06 | 11.58800 | 0.56974 | 2.54243 | 2 |
| **HSC** | 7.35E-06 | 11.04994 | 0.51806 | 2.43051 | 2 |
| **MSC** | 0.000101 | 18.86202 | 1.75759 | 4.60332 | 3 |
| **MSC** | 0.000116 | 21.87775 | 2.36453 | 5.30138 | 3 |
| **MSC** | 0.000117 | 22.08720 | 2.41003 | 5.34925 | 3 |
| **MSC** | 0.000119 | 22.34743 | 2.46715 | 5.40861 | 3 |
| **MSC** | 0.00012 | 22.58901 | 2.52078 | 5.46360 | 3 |
| **MSC** | 0.000142 | 27.06451 | 3.61860 | 6.46016 | 3 |
| **FIB** | 0.002312 | 0.31267 | 0.00227 | 0.10960 | 1 |
| **FIB** | 0.003014 | 1.71883 | 0.06851 | 0.60092 | 1 |
| **FIB** | 0.003032 | 1.75397 | 0.07134 | 0.61314 | 1 |
| **FIB** | 0.003514 | 2.63203 | 0.16065 | 0.91685 | 1 |
| **FIB** | 0.003565 | 2.72294 | 0.17194 | 0.94809 | 1 |
| **FIB** | 0.003808 | 3.14472 | 0.22933 | 1.09245 | 1 |
| **FIB** | 0.004359 | 4.07451 | 0.38499 | 1.40663 | 1 |
| **FIB** | 0.004504 | 4.31537 | 0.43185 | 1.48696 | 1 |
| **HSC** | 0.999996 | -2.18530 | 0.02026 | 0.49221 | 2 |

| **COL4A2** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **MSC** | 8.02E-14 | 0.11975 | 0.00001 | 0.01623 | 3 |
| **MSC** | 8.55E-14 | 1.86879 | 0.00253 | 0.25323 | 3 |
| **MSC** | 8.68E-14 | 2.33470 | 0.00395 | 0.31635 | 3 |
| **MSC** | 8.77E-14 | 2.66716 | 0.00515 | 0.36137 | 3 |
| **MSC** | 8.79E-14 | 2.75443 | 0.00549 | 0.37319 | 3 |
| **MSC** | 8.80E-14 | 2.77724 | 0.00558 | 0.37628 | 3 |
| **MSC** | 8.89E-14 | 3.14449 | 0.00716 | 0.42601 | 3 |
| **MSC** | 8.94E-14 | 3.35154 | 0.00813 | 0.45404 | 3 |
| **MSC** | 8.96E-14 | 3.43214 | 0.00853 | 0.46495 | 3 |
| **MSC** | 9.20E-14 | 4.45171 | 0.01435 | 0.60290 | 3 |
| **MSC** | 9.35E-14 | 5.21560 | 0.01969 | 0.70618 | 3 |
| **MSC** | 9.44E-14 | 5.66241 | 0.02321 | 0.76655 | 3 |
| **MSC** | 9.52E-14 | 15.22992 | 0.16793 | 2.04776 | 3 |
| **MSC** | 9.60E-14 | 14.72275 | 0.15693 | 1.98060 | 3 |
| **MSC** | 9.63E-14 | 14.55458 | 0.15337 | 1.95831 | 3 |
| **MSC** | 9.63E-14 | 14.50042 | 0.15223 | 1.95113 | 3 |
| **MSC** | 9.66E-14 | 14.34126 | 0.14890 | 1.93002 | 3 |
| **MSC** | 9.66E-14 | 7.12982 | 0.03680 | 0.96459 | 3 |
| **MSC** | 9.67E-14 | 14.23674 | 0.14674 | 1.91615 | 3 |
| **MSC** | 9.71E-14 | 13.94264 | 0.14074 | 1.87710 | 3 |
| **MSC** | 9.71E-14 | 7.47371 | 0.04044 | 1.01095 | 3 |
| **MSC** | 9.71E-14 | 13.89104 | 0.13970 | 1.87024 | 3 |
| **MSC** | 9.72E-14 | 13.81143 | 0.13810 | 1.85966 | 3 |
| **MSC** | 9.72E-14 | 13.79559 | 0.13779 | 1.85756 | 3 |
| **MSC** | 9.73E-14 | 13.72065 | 0.13629 | 1.84760 | 3 |
| **MSC** | 9.77E-14 | 13.38056 | 0.12962 | 1.80237 | 3 |
| **MSC** | 9.77E-14 | 13.30431 | 0.12815 | 1.79222 | 3 |
| **MSC** | 9.78E-14 | 13.23098 | 0.12674 | 1.78246 | 3 |
| **MSC** | 9.79E-14 | 8.29991 | 0.04987 | 1.12221 | 3 |
| **MSC** | 9.79E-14 | 8.30931 | 0.04999 | 1.12347 | 3 |
| **MSC** | 9.82E-14 | 8.62488 | 0.05386 | 1.16593 | 3 |
| **MSC** | 9.83E-14 | 12.59005 | 0.11476 | 1.69708 | 3 |
| **MSC** | 9.85E-14 | 12.32932 | 0.11005 | 1.66230 | 3 |
| **MSC** | 9.88E-14 | 11.77876 | 0.10045 | 1.58879 | 3 |
| **MSC** | 9.88E-14 | 9.74757 | 0.06879 | 1.31677 | 3 |
| **MSC** | 9.89E-14 | 11.49349 | 0.09564 | 1.55066 | 3 |
| **MSC** | 9.89E-14 | 10.23586 | 0.07585 | 1.38227 | 3 |
| **MSC** | 9.90E-14 | 11.11588 | 0.08946 | 1.50015 | 3 |
| **MSC** | 9.90E-14 | 11.09981 | 0.08920 | 1.49800 | 3 |
| **MSC** | 9.90E-14 | 10.99741 | 0.08756 | 1.48430 | 3 |
| **MSC** | 9.90E-14 | 10.99286 | 0.08749 | 1.48369 | 3 |
| **MSC** | 9.90E-14 | 10.91793 | 0.08630 | 1.47366 | 3 |
| **MSC** | 9.90E-14 | 10.62653 | 0.08175 | 1.43463 | 3 |
| **MSC** | 9.90E-14 | 10.78944 | 0.08428 | 1.45645 | 3 |
| **MSC** | 9.90E-14 | 10.74003 | 0.08351 | 1.44984 | 3 |
| **HSC** | 6.24E-13 | 38.53963 | 2.12487 | 5.12882 | 2 |
| **HSC** | 6.29E-13 | 38.79649 | 2.15329 | 5.16185 | 2 |
| **HSC** | 6.83E-13 | 41.40141 | 2.45216 | 5.49554 | 2 |
| **HSC** | 7.83E-13 | 46.42067 | 3.08277 | 6.13117 | 2 |
| **HSC** | 8.06E-13 | 47.64827 | 3.24797 | 6.28505 | 2 |
| **HSC** | 8.36E-13 | 49.32172 | 3.48012 | 6.49375 | 2 |
| **HSC** | 8.46E-13 | 49.88070 | 3.55945 | 6.56318 | 2 |
| **HSC** | 8.69E-13 | 51.27581 | 3.76134 | 6.73584 | 2 |
| **HSC** | 8.71E-13 | 51.40013 | 3.77960 | 6.75118 | 2 |
| **HSC** | 8.90E-13 | 52.62325 | 3.96162 | 6.90173 | 2 |
| **FIB** | 1.75E-08 | 1.57001 | 0.01156 | 0.28260 | 1 |
| **FIB** | 2.20E-08 | 3.25440 | 0.04966 | 0.58545 | 1 |
| **FIB** | 2.23E-08 | 3.36305 | 0.05303 | 0.60496 | 1 |
| **FIB** | 3.01E-08 | 5.73001 | 0.15395 | 1.02913 | 1 |
| **FIB** | 3.10E-08 | 5.96703 | 0.16695 | 1.07148 | 1 |
| **FIB** | 3.52E-08 | 7.07303 | 0.23458 | 1.26874 | 1 |
| **FIB** | 5.82E-08 | 11.91181 | 0.66533 | 2.12230 | 1 |
| **FIB** | 7.58E-08 | 14.98464 | 1.05286 | 2.65336 | 1 |
| **FIB** | 7.61E-08 | 15.04206 | 1.06095 | 2.66318 | 1 |
| **FIB** | 7.70E-08 | 15.17623 | 1.07996 | 2.68612 | 1 |
| **FIB** | 1.04E-07 | 19.52660 | 1.78786 | 3.41667 | 1 |
| **MSC** | 1 | -0.07991 | 0.00000 | 0.01083 | 3 |
| **MSC** | 1 | -1.38671 | 0.00139 | 0.18792 | 3 |
| **MSC** | 1 | -5.45979 | 0.02158 | 0.73918 | 3 |
| **MSC** | 1 | -6.33494 | 0.02905 | 0.85736 | 3 |

| | **DT2: Exon Arrays (N=15)** | | | | |
|---|---|---|---|---|---|
| **Cell-types** | **p-value** | **Statistic** | **Expected** | **Std.dev** | **Residual** |
| **MSC** | 0.000391 | 8.22205 | 0.70488 | 2.23805 | 3 |
| **MSC** | 0.000392 | 1.85923 | 0.03604 | 0.54286 | 3 |
| **MSC** | 0.000421 | 7.30685 | 0.55669 | 2.02184 | 3 |
| **MSC** | 0.000424 | 7.20095 | 0.54067 | 1.99602 | 3 |
| **MSC** | 0.000427 | 3.11615 | 0.10125 | 0.90403 | 3 |
| **MSC** | 0.000429 | 3.22678 | 0.10857 | 0.93544 | 3 |
| **MSC** | 0.00043 | 3.29452 | 0.11317 | 0.95465 | 3 |
| **MSC** | 0.000441 | 6.36768 | 0.42278 | 1.78749 | 3 |
| **HSC** | 0.002618 | 7.79036 | 1.35433 | 2.30503 | 2 |
| **HSC** | 0.003001 | 8.99077 | 1.80386 | 2.61559 | 2 |
| **HSC** | 0.003851 | 11.99496 | 3.21075 | 3.29629 | 2 |
| **FIB** | 0.021283 | 1.64883 | 0.23312 | 0.69810 | 1 |
| **FIB** | 0.023834 | 1.94650 | 0.32490 | 0.81886 | 1 |
| **FIB** | 0.025228 | 2.10189 | 0.37884 | 0.88087 | 1 |
| **MSC** | 0.999776 | -2.23179 | 0.05194 | 0.65062 | 3 |

| **DT3: RNA-seq (N=29)** | | | | | |
|---|---|---|---|---|---|
| **Cell-types** | **p-value** | **Statistic** | **Expected** | **Std.dev** | **Residual** |
| **HSC** | 1.58E-06 | 0.06389 | 0.00002 | 0.01370 | 2 |
| **HSC** | 1.68E-06 | 20.38177 | 1.56459 | 4.04856 | 2 |
| **HSC** | 1.68E-06 | 0.84317 | 0.00268 | 0.18085 | 2 |
| **HSC** | 1.79E-06 | 1.69139 | 0.01077 | 0.36265 | 2 |
| **HSC** | 2.11E-06 | 17.26240 | 1.12233 | 3.50858 | 2 |
| **HSC** | 2.35E-06 | 6.92295 | 0.18051 | 1.47280 | 2 |
| **HSC** | 2.37E-06 | 7.29892 | 0.20065 | 1.55133 | 2 |
| **HSC** | 2.38E-06 | 14.47953 | 0.78964 | 2.99223 | 2 |
| **HSC** | 2.45E-06 | 8.61236 | 0.27936 | 1.82380 | 2 |
| **HSC** | 2.46E-06 | 13.11109 | 0.64743 | 2.72829 | 2 |
| **HSC** | 2.48E-06 | 9.27309 | 0.32387 | 1.95963 | 2 |
| **HSC** | 2.48E-06 | 9.41185 | 0.33363 | 1.98804 | 2 |
| **HSC** | 2.50E-06 | 11.49002 | 0.49723 | 2.40828 | 2 |
| **HSC** | 2.50E-06 | 10.85812 | 0.44405 | 2.28160 | 2 |
| **MSC** | 0.000242 | 18.16257 | 1.78995 | 4.69159 | 3 |
| **MSC** | 0.000245 | 18.45673 | 1.84840 | 4.76438 | 3 |
| **MSC** | 0.000251 | 18.90297 | 1.93886 | 4.87452 | 3 |
| **MSC** | 0.000251 | 18.95333 | 1.94921 | 4.88692 | 3 |
| **MSC** | 0.000265 | 20.10800 | 2.19394 | 5.17006 | 3 |
| **MSC** | 0.000307 | 23.49977 | 2.99650 | 5.98588 | 3 |
| **FIB** | 0.000672 | 1.88887 | 0.06045 | 0.57023 | 1 |
| **FIB** | 0.000763 | 2.67503 | 0.12123 | 0.80567 | 1 |
| **FIB** | 0.000807 | 3.04447 | 0.15703 | 0.91567 | 1 |
| **FIB** | 0.000865 | 3.52124 | 0.21006 | 1.05687 | 1 |
| **FIB** | 0.000892 | 3.73507 | 0.23635 | 1.11990 | 1 |
| **FIB** | 0.001012 | 4.68681 | 0.37215 | 1.39778 | 1 |
| **FIB** | 0.001127 | 5.58892 | 0.52920 | 1.65644 | 1 |
| **FIB** | 0.00113 | 5.61337 | 0.53384 | 1.66338 | 1 |
| **HSC** | 0.999999 | -2.18845 | 0.01804 | 0.46906 | 2 |

| **SCUBE3** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **MSC** | 1.62E-07 | 1.92408 | 0.00365 | 0.37593 | 3 |
| **MSC** | 1.64E-07 | 2.23736 | 0.00493 | 0.43712 | 3 |
| **MSC** | 1.64E-07 | 2.35783 | 0.00547 | 0.46065 | 3 |
| **MSC** | 1.65E-07 | 29.81512 | 0.87541 | 5.66814 | 3 |
| **MSC** | 1.66E-07 | 2.90681 | 0.00832 | 0.56786 | 3 |
| **MSC** | 1.73E-07 | 4.79418 | 0.02263 | 0.93615 | 3 |
| **MSC** | 1.74E-07 | 27.16250 | 0.72657 | 5.18858 | 3 |
| **MSC** | 1.78E-07 | 25.85190 | 0.65815 | 4.94901 | 3 |
| **MSC** | 1.79E-07 | 7.08276 | 0.04940 | 1.38191 | 3 |
| **MSC** | 1.80E-07 | 25.21709 | 0.62622 | 4.83239 | 3 |
| **MSC** | 1.82E-07 | 24.38017 | 0.58534 | 4.67807 | 3 |
| **MSC** | 1.85E-07 | 9.30586 | 0.08528 | 1.81366 | 3 |
| **MSC** | 1.86E-07 | 22.84763 | 0.51407 | 4.39389 | 3 |
| **MSC** | 1.87E-07 | 10.56618 | 0.10994 | 2.05773 | 3 |
| **MSC** | 1.89E-07 | 11.30815 | 0.12593 | 2.20114 | 3 |
| **MSC** | 1.89E-07 | 11.54069 | 0.13116 | 2.24605 | 3 |
| **MSC** | 1.89E-07 | 11.68822 | 0.13453 | 2.27452 | 3 |
| **MSC** | 1.89E-07 | 20.97465 | 0.43324 | 4.04395 | 3 |
| **MSC** | 1.89E-07 | 20.92662 | 0.43126 | 4.03494 | 3 |
| **MSC** | 1.89E-07 | 11.80059 | 0.13713 | 2.29620 | 3 |
| **MSC** | 1.90E-07 | 12.35469 | 0.15031 | 2.40305 | 3 |
| **MSC** | 1.90E-07 | 20.30323 | 0.40595 | 3.91785 | 3 |
| **MSC** | 1.90E-07 | 20.24921 | 0.40379 | 3.90769 | 3 |
| **MSC** | 1.90E-07 | 20.10622 | 0.39811 | 3.88079 | 3 |
| **MSC** | 1.91E-07 | 13.20374 | 0.17168 | 2.56650 | 3 |
| **MSC** | 1.91E-07 | 13.70079 | 0.18485 | 2.66203 | 3 |
| **MSC** | 1.92E-07 | 18.83372 | 0.34931 | 3.64072 | 3 |
| **MSC** | 1.92E-07 | 14.21228 | 0.19891 | 2.76022 | 3 |
| **MSC** | 1.92E-07 | 15.26666 | 0.22952 | 2.96219 | 3 |
| **MSC** | 1.92E-07 | 15.44542 | 0.23493 | 2.99637 | 3 |
| **FIB** | 2.59E-07 | 7.43264 | 0.19744 | 1.44138 | 1 |
| **FIB** | 3.14E-07 | 9.88486 | 0.34922 | 1.91396 | 1 |
| **FIB** | 3.81E-07 | 12.46160 | 0.55501 | 2.40781 | 1 |
| **FIB** | 3.83E-07 | 12.54483 | 0.56245 | 2.42371 | 1 |
| **FIB** | 4.06E-07 | 13.35739 | 0.63767 | 2.57870 | 1 |
| **FIB** | 4.12E-07 | 13.58012 | 0.65912 | 2.62113 | 1 |
| **FIB** | 4.69E-07 | 15.50460 | 0.85916 | 2.98642 | 1 |
| **FIB** | 4.84E-07 | 15.96983 | 0.91150 | 3.07437 | 1 |
| **FIB** | 9.42E-07 | 28.37094 | 2.87675 | 5.34972 | 1 |
| **FIB** | 9.46E-07 | 28.47092 | 2.89706 | 5.36740 | 1 |
| **FIB** | 1.12E-06 | 32.91358 | 3.87173 | 6.13947 | 1 |
| **HSC** | 0.0002263 | 11.92978 | 0.54216 | 3.24685 | 2 |
| **HSC** | 0.0002299 | 12.26740 | 0.57328 | 3.33822 | 2 |
| **HSC** | 0.0002531 | 14.35977 | 0.78552 | 3.90346 | 2 |
| **HSC** | 0.0003221 | 19.88827 | 1.50680 | 5.38679 | 2 |
| **HSC** | 0.0003370 | 20.97038 | 1.67523 | 5.67507 | 2 |
| **HSC** | 0.0003850 | 24.25177 | 2.24053 | 6.54438 | 2 |
| **HSC** | 0.0004919 | 30.68506 | 3.58688 | 8.22376 | 2 |
| **HSC** | 0.9999017 | -4.12942 | 0.06496 | 1.12654 | 2 |
| **HSC** | 0.9999077 | -5.22909 | 0.10416 | 1.42627 | 2 |
| **HSC** | 0.9999139 | -6.39822 | 0.15595 | 1.74471 | 2 |
| **MSC** | 0.9999998 | -0.84174 | 0.00070 | 0.16447 | 3 |
| **MSC** | 0.9999999 | -2.11767 | 0.00442 | 0.41374 | 3 |
| **MSC** | 0.9999999 | -2.14979 | 0.00455 | 0.42002 | 3 |
| **MSC** | 0.9999999 | -2.44614 | 0.00589 | 0.47790 | 3 |
| **MSC** | 0.9999999 | -2.80253 | 0.00773 | 0.54749 | 3 |
| **MSC** | 0.9999999 | -4.32052 | 0.01838 | 0.84377 | 3 |
| **MSC** | 0.9999999 | -5.06805 | 0.02529 | 0.98955 | 3 |
| **MSC** | 0.9999999 | -5.78429 | 0.03295 | 1.12913 | 3 |
| **MSC** | 0.9999999 | -6.61075 | 0.04304 | 1.29007 | 3 |
| **MSC** | 0.9999999 | -7.24833 | 0.05174 | 1.41411 | 3 |
| **MSC** | 0.9999999 | -8.90449 | 0.07808 | 1.73582 | 3 |
| **MSC** | 0.9999999 | -8.92729 | 0.07848 | 1.74024 | 3 |
| **MSC** | 0.9999999 | -9.10764 | 0.08169 | 1.77522 | 3 |
| **MSC** | 0.9999999 | -9.38880 | 0.08681 | 1.82974 | 3 |
| **MSC** | 0.9999999 | -10.53328 | 0.10926 | 2.05136 | 3 |
| **MSC** | 0.9999999 | -10.55442 | 0.10970 | 2.05545 | 3 |
| **MSC** | 0.9999999 | -11.44440 | 0.12898 | 2.22745 | 3 |
| **MSC** | 0.9999999 | -12.36802 | 0.15064 | 2.40562 | 3 |
| **MSC** | 1.0000000 | -24.70613 | 0.60110 | 4.73825 | 3 |

| | **DT2: Exon Arrays (N=15)** | | | | |
|---|---|---|---|---|---|
| **Cell-types** | **p-value** | **Statistic** | **Expected** | **Std.dev** | **Residual** |
| **MSC** | 0.000287 | 11.01684 | 1.18760 | 2.85423 | 3 |
| **MSC** | 0.000366 | 1.29528 | 0.01642 | 0.37865 | 3 |
| **MSC** | 0.000409 | 8.05917 | 0.63553 | 2.21832 | 3 |
| **MSC** | 0.000442 | 6.23567 | 0.38047 | 1.76105 | 3 |
| **MSC** | 0.000445 | 5.06187 | 0.25071 | 1.44764 | 3 |
| **MSC** | 0.000445 | 5.60862 | 0.30780 | 1.59521 | 3 |
| **MSC** | 0.000445 | 5.43616 | 0.28916 | 1.54895 | 3 |
| **FIB** | 0.008527 | 3.21226 | 0.57428 | 1.10581 | 1 |
| **FIB** | 0.008827 | 3.33663 | 0.61961 | 1.14507 | 1 |
| **HSC** | 0.008909 | 5.78411 | 0.96079 | 2.03569 | 2 |
| **FIB** | 0.010224 | 3.89747 | 0.84541 | 1.31666 | 1 |
| **HSC** | 0.010533 | 6.96583 | 1.39348 | 2.41562 | 2 |
| **HSC** | 0.010926 | 7.24122 | 1.50584 | 2.50132 | 2 |
| **MSC** | 0.999693 | -0.28729 | 0.00081 | 0.08410 | 3 |
| **MSC** | 0.999744 | -1.54698 | 0.02342 | 0.45194 | 3 |

| **DT3: RNA-seq (N=29)** | | | | | |
|---|---|---|---|---|---|
| **Cell-types** | **p-value** | **Statistic** | **Expected** | **Std.dev** | **Residual** |
| **MSC** | 6.89E-06 | 21.44480 | 2.31344 | 4.40079 | 3 |
| **MSC** | 7.55E-06 | 23.99602 | 2.89663 | 4.87584 | 3 |
| **MSC** | 7.56E-06 | 24.04753 | 2.90908 | 4.88527 | 3 |
| **MSC** | 7.63E-06 | 24.36518 | 2.98644 | 4.94323 | 3 |
| **MSC** | 7.83E-06 | 25.27382 | 3.21334 | 5.10753 | 3 |
| **MSC** | 7.90E-06 | 38.06094 | 7.28742 | 7.12805 | 3 |
| **HSC** | 0.0106310 | 0.30801 | 0.00205 | 0.13284 | 2 |
| **HSC** | 0.0113420 | 0.83732 | 0.01515 | 0.36080 | 2 |
| **HSC** | 0.0119219 | 1.27643 | 0.03520 | 0.54931 | 2 |
| **HSC** | 0.0119496 | 1.29759 | 0.03638 | 0.55837 | 2 |
| **HSC** | 0.0121410 | 1.44468 | 0.04509 | 0.62131 | 2 |
| **HSC** | 0.0122552 | 1.53292 | 0.05077 | 0.65902 | 2 |
| **HSC** | 0.0124603 | 1.69264 | 0.06190 | 0.72716 | 2 |
| **HSC** | 0.0125299 | 1.74721 | 0.06595 | 0.75040 | 2 |
| **HSC** | 0.0125355 | 1.75158 | 0.06628 | 0.75226 | 2 |
| **HSC** | 0.0131161 | 2.21500 | 0.10600 | 0.94882 | 2 |
| **HSC** | 0.0135018 | 2.53260 | 0.13857 | 1.08255 | 2 |
| **HSC** | 0.0136638 | 2.66878 | 0.15387 | 1.13961 | 2 |
| **HSC** | 0.0138792 | 2.85283 | 0.17583 | 1.21644 | 2 |
| **HSC** | 0.0146075 | 3.50581 | 0.26553 | 1.48598 | 2 |
| **FIB** | 0.4325343 | 0.29635 | 0.16346 | 0.78203 | 1 |
| **FIB** | 0.4875218 | 0.49363 | 0.45354 | 1.28151 | 1 |
| **FIB** | 0.4958380 | 0.52276 | 0.50865 | 1.35284 | 1 |
| **FIB** | 0.5090163 | 0.56852 | 0.60160 | 1.46336 | 1 |
| **FIB** | 0.6487674 | -0.00921 | 0.00016 | 0.02453 | 1 |
| **FIB** | 0.6651548 | -0.07266 | 0.00983 | 0.19338 | 1 |
| **FIB** | 0.6898222 | -0.16931 | 0.05336 | 0.44953 | 1 |
| **FIB** | 0.6915477 | -0.17613 | 0.05774 | 0.46750 | 1 |
| **HSC** | 0.9908796 | -0.80165 | 0.01388 | 0.34546 | 2 |

| **ENG(CD105)** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **HSC** | 2.93E-08 | 13.65221 | 0.31635 | 2.45916 | 2 |
| **HSC** | 3.49E-08 | 17.56086 | 0.52343 | 3.15990 | 2 |
| **HSC** | 3.89E-08 | 20.13093 | 0.68785 | 3.61933 | 2 |
| **HSC** | 9.79E-08 | 46.36202 | 3.64832 | 8.20901 | 2 |
| **HSC** | 1.15E-07 | 52.18212 | 4.62181 | 9.19227 | 2 |
| **HSC** | 1.17E-07 | 52.90456 | 4.75067 | 9.31318 | 2 |
| **HSC** | 1.20E-07 | 54.11098 | 4.96980 | 9.51448 | 2 |
| **HSC** | 1.21E-07 | 54.47377 | 5.03667 | 9.57486 | 2 |
| **HSC** | 1.23E-07 | 54.87360 | 5.11087 | 9.64133 | 2 |
| **HSC** | 1.48E-07 | 63.36060 | 6.81407 | 11.03112 | 2 |
| **MSC** | 4.48E-07 | 0.21864 | 0.00005 | 0.04449 | 3 |
| **MSC** | 4.65E-07 | 1.63492 | 0.00291 | 0.33265 | 3 |
| **MSC** | 4.80E-07 | 28.77361 | 0.90131 | 5.68840 | 3 |
| **MSC** | 4.84E-07 | 3.40041 | 0.01259 | 0.69166 | 3 |
| **MSC** | 4.87E-07 | 28.09477 | 0.85928 | 5.56189 | 3 |
| **MSC** | 4.87E-07 | 3.72963 | 0.01514 | 0.75857 | 3 |
| **MSC** | 4.88E-07 | 3.83844 | 0.01604 | 0.78068 | 3 |
| **MSC** | 4.92E-07 | 4.21466 | 0.01934 | 0.85711 | 3 |
| **MSC** | 4.94E-07 | 4.43317 | 0.02140 | 0.90148 | 3 |
| **MSC** | 4.95E-07 | 4.46183 | 0.02167 | 0.90730 | 3 |
| **MSC** | 4.95E-07 | 27.29074 | 0.81080 | 5.41132 | 3 |
| **MSC** | 4.96E-07 | 4.65562 | 0.02360 | 0.94666 | 3 |
| **MSC** | 4.97E-07 | 4.75639 | 0.02463 | 0.96711 | 3 |
| **MSC** | 5.00E-07 | 5.07476 | 0.02804 | 1.03174 | 3 |
| **MSC** | 5.01E-07 | 5.15513 | 0.02893 | 1.04805 | 3 |
| **MSC** | 5.04E-07 | 5.47975 | 0.03269 | 1.11392 | 3 |
| **MSC** | 5.05E-07 | 26.26610 | 0.75106 | 5.21834 | 3 |
| **MSC** | 5.06E-07 | 26.19776 | 0.74716 | 5.20543 | 3 |
| **MSC** | 5.06E-07 | 5.72705 | 0.03571 | 1.16408 | 3 |
| **MSC** | 5.07E-07 | 5.78689 | 0.03646 | 1.17621 | 3 |
| **MSC** | 5.11E-07 | 6.28702 | 0.04303 | 1.27760 | 3 |
| **MSC** | 5.12E-07 | 25.53903 | 0.71006 | 5.08068 | 3 |
| **MSC** | 5.12E-07 | 6.43379 | 0.04506 | 1.30735 | 3 |
| **MSC** | 5.23E-07 | 7.73367 | 0.06511 | 1.57050 | 3 |
| **MSC** | 5.29E-07 | 23.39845 | 0.59602 | 4.67209 | 3 |
| **MSC** | 5.29E-07 | 23.33516 | 0.59280 | 4.65994 | 3 |
| **MSC** | 5.30E-07 | 23.17484 | 0.58468 | 4.62914 | 3 |
| **MSC** | 5.31E-07 | 8.96039 | 0.08741 | 1.81834 | 3 |
| **MSC** | 5.37E-07 | 9.84489 | 0.10551 | 1.99670 | 3 |
| **MSC** | 5.38E-07 | 10.15983 | 0.11237 | 2.06014 | 3 |
| **MSC** | 5.45E-07 | 11.43580 | 0.14237 | 2.31669 | 3 |
| **MSC** | 5.50E-07 | 12.98137 | 0.18345 | 2.62640 | 3 |
| **MSC** | 5.52E-07 | 18.41842 | 0.36931 | 3.70455 | 3 |
| **MSC** | 5.55E-07 | 16.06296 | 0.28089 | 3.23988 | 3 |
| **FIB** | 0.0010614 | 2.65386 | 0.06842 | 0.84148 | 1 |
| **FIB** | 0.0011478 | 3.46868 | 0.11689 | 1.09929 | 1 |
| **FIB** | 0.0011586 | 3.56756 | 0.12365 | 1.13055 | 1 |
| **FIB** | 0.0012616 | 4.47868 | 0.19487 | 1.41823 | 1 |
| **FIB** | 0.0012675 | 4.52846 | 0.19922 | 1.43393 | 1 |
| **FIB** | 0.0012810 | 4.64429 | 0.20954 | 1.47045 | 1 |
| **FIB** | 0.0013136 | 4.91825 | 0.23499 | 1.55678 | 1 |
| **FIB** | 0.0013442 | 5.17204 | 0.25987 | 1.63669 | 1 |
| **FIB** | 0.0014460 | 5.98748 | 0.34828 | 1.89299 | 1 |
| **FIB** | 0.9992236 | -0.42524 | 0.00176 | 0.13493 | 1 |
| **FIB** | 0.9994068 | -2.87014 | 0.08003 | 0.90995 | 1 |
| **MSC** | 0.9999996 | -1.80648 | 0.00355 | 0.36755 | 3 |
| **MSC** | 0.9999996 | -1.94585 | 0.00412 | 0.39590 | 3 |
| **MSC** | 0.9999996 | -2.51037 | 0.00686 | 0.51072 | 3 |
| **MSC** | 0.9999996 | -2.78690 | 0.00846 | 0.56695 | 3 |
| **MSC** | 0.9999996 | -3.97746 | 0.01722 | 0.80892 | 3 |
| **MSC** | 0.9999996 | -6.52547 | 0.04636 | 1.32592 | 3 |
| **MSC** | 0.9999997 | -8.34336 | 0.07578 | 1.69374 | 3 |
| **MSC** | 0.9999997 | -10.14063 | 0.11195 | 2.05627 | 3 |
| **MSC** | 0.9999997 | -10.24705 | 0.11431 | 2.07769 | 3 |
| **MSC** | 0.9999997 | -11.57381 | 0.14583 | 2.34439 | 3 |
| **MSC** | 0.9999997 | -11.65353 | 0.14784 | 2.36039 | 3 |
| **MSC** | 0.9999997 | -12.11227 | 0.15971 | 2.45239 | 3 |
| **MSC** | 0.9999997 | -12.49305 | 0.16991 | 2.52867 | 3 |
| **MSC** | 0.9999997 | -13.09026 | 0.18654 | 2.64817 | 3 |
| **MSC** | 0.9999998 | -15.52548 | 0.26241 | 3.13330 | 3 |

| | **DT2: Exon Arrays (N=15)** | | | | |
|---|---|---|---|---|---|
| **Cell-types** | **p-value** | **Statistic** | **Expected** | **Std.dev** | **Residual** |
| **MSC** | 0.0012322 | 11.39211 | 1.72498 | 3.19292 | 3 |
| **MSC** | 0.0018249 | 1.10299 | 0.01617 | 0.37387 | 3 |
| **MSC** | 0.0019090 | 1.57896 | 0.03314 | 0.53437 | 3 |
| **MSC** | 0.0020853 | 2.74235 | 0.09996 | 0.92231 | 3 |
| **MSC** | 0.0021792 | 7.17876 | 0.68498 | 2.27773 | 3 |
| **MSC** | 0.0022003 | 3.79735 | 0.19166 | 1.26608 | 3 |
| **MSC** | 0.0022303 | 4.19287 | 0.23367 | 1.39232 | 3 |
| **HSC** | 0.0033635 | 9.80247 | 2.06577 | 2.85480 | 2 |
| **HSC** | 0.0038015 | 11.21613 | 2.70456 | 3.18880 | 2 |
| **HSC** | 0.0038170 | 11.26895 | 2.73009 | 3.20066 | 2 |
| **FIB** | 0.0687687 | 0.38192 | 0.02544 | 0.24005 | 1 |
| **FIB** | 0.0840365 | 0.79298 | 0.10967 | 0.49572 | 1 |
| **FIB** | 0.0840833 | 0.79417 | 0.11000 | 0.49645 | 1 |
| **MSC** | 0.9984892 | -0.48249 | 0.00309 | 0.16374 | 3 |
| **MSC** | 0.9986863 | -1.40865 | 0.02637 | 0.47703 | 3 |

| **DT3: RNA-seq (N=29)** | | | | | |
|---|---|---|---|---|---|
| **Cell-types** | **p-value** | **Statistic** | **Expected** | **Std.dev** | **Residual** |
| **HSC** | 2.59E-05 | 0.64439 | 0.00244 | 0.15861 | 2 |
| **HSC** | 3.16E-05 | 3.08168 | 0.05569 | 0.75636 | 2 |
| **HSC** | 3.22E-05 | 14.78862 | 1.28252 | 3.37984 | 2 |
| **HSC** | 3.32E-05 | 3.84741 | 0.08681 | 0.94271 | 2 |
| **HSC** | 3.32E-05 | 3.87843 | 0.08821 | 0.95024 | 2 |
| **HSC** | 3.35E-05 | 4.02863 | 0.09517 | 0.98666 | 2 |
| **HSC** | 3.38E-05 | 4.17768 | 0.10235 | 1.02277 | 2 |
| **HSC** | 3.39E-05 | 4.24473 | 0.10566 | 1.03899 | 2 |
| **HSC** | 3.43E-05 | 4.46922 | 0.11713 | 1.09326 | 2 |
| **HSC** | 3.50E-05 | 4.82771 | 0.13668 | 1.17969 | 2 |
| **HSC** | 3.51E-05 | 4.87784 | 0.13953 | 1.19176 | 2 |
| **HSC** | 3.73E-05 | 6.45402 | 0.24427 | 1.56779 | 2 |
| **HSC** | 3.74E-05 | 6.50659 | 0.24827 | 1.58022 | 2 |
| **HSC** | 3.75E-05 | 6.59596 | 0.25513 | 1.60131 | 2 |
| **HSC** | 3.86E-05 | 10.65083 | 0.66523 | 2.52632 | 2 |
| **MSC** | 0.0006459 | 0.93274 | 0.00764 | 0.28750 | 3 |
| **MSC** | 0.0011655 | 7.74401 | 0.52630 | 2.37078 | 3 |
| **MSC** | 0.0023165 | 18.41079 | 2.97471 | 5.45155 | 3 |
| **FIB** | 0.0023913 | 1.47461 | 0.05562 | 0.50295 | 1 |
| **MSC** | 0.0025376 | 20.21762 | 3.58723 | 5.93471 | 3 |
| **FIB** | 0.0028762 | 2.43288 | 0.15138 | 0.82616 | 1 |
| **FIB** | 0.0030259 | 2.71590 | 0.18865 | 0.92068 | 1 |
| **FIB** | 0.0034997 | 3.58557 | 0.32882 | 1.20760 | 1 |
| **MSC** | 0.0038089 | 31.48539 | 8.69996 | 8.53849 | 3 |
| **FIB** | 0.0044053 | 5.19591 | 0.69050 | 1.72007 | 1 |
| **FIB** | 0.0050444 | 6.35611 | 1.03329 | 2.06891 | 1 |
| **FIB** | 0.9983627 | -0.21479 | 0.00118 | 0.07344 | 1 |
| **FIB** | 0.9986375 | -0.93119 | 0.02218 | 0.31809 | 1 |
| **MSC** | 0.9997092 | -6.33057 | 0.35171 | 1.94251 | 3 |

| **THY1(CD90)** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **HSC** | 4.04E-10 | 33.64927 | 2.49040 | 5.07206 | 2 |
| **HSC** | 4.07E-10 | 33.80732 | 2.51385 | 5.09477 | 2 |
| **HSC** | 4.25E-10 | 35.02282 | 2.69786 | 5.26883 | 2 |
| **HSC** | 5.20E-10 | 41.52435 | 3.79248 | 6.18225 | 2 |
| **HSC** | 5.28E-10 | 42.19084 | 3.91520 | 6.27407 | 2 |
| **HSC** | 5.62E-10 | 44.96584 | 4.44716 | 6.65239 | 2 |
| **HSC** | 5.77E-10 | 46.43226 | 4.74195 | 6.84961 | 2 |
| **HSC** | 5.79E-10 | 46.67344 | 4.79134 | 6.88186 | 2 |
| **HSC** | 5.95E-10 | 48.46114 | 5.16541 | 7.11922 | 2 |
| **HSC** | 6.05E-10 | 49.77531 | 5.44936 | 7.29177 | 2 |
| **MSC** | 0.0003855 | 0.10337 | 0.00004 | 0.03072 | 3 |
| **MSC** | 0.0003924 | 0.47024 | 0.00091 | 0.13976 | 3 |
| **MSC** | 0.0003998 | 0.87364 | 0.00314 | 0.25962 | 3 |
| **MSC** | 0.0004001 | 0.88641 | 0.00324 | 0.26342 | 3 |
| **MSC** | 0.0004005 | 0.90797 | 0.00340 | 0.26982 | 3 |
| **MSC** | 0.0004011 | 0.94126 | 0.00365 | 0.27971 | 3 |
| **MSC** | 0.0004063 | 1.23365 | 0.00627 | 0.36655 | 3 |
| **MSC** | 0.0004088 | 1.37777 | 0.00782 | 0.40933 | 3 |
| **MSC** | 0.0004124 | 1.58961 | 0.01041 | 0.47220 | 3 |
| **MSC** | 0.0004132 | 1.63523 | 0.01102 | 0.48574 | 3 |
| **MSC** | 0.0004198 | 2.03342 | 0.01703 | 0.60382 | 3 |
| **MSC** | 0.0004245 | 2.32880 | 0.02234 | 0.69133 | 3 |
| **MSC** | 0.0004261 | 2.42935 | 0.02431 | 0.72110 | 3 |
| **MSC** | 0.0004328 | 2.86881 | 0.03391 | 0.85109 | 3 |
| **MSC** | 0.0004346 | 2.98997 | 0.03683 | 0.88689 | 3 |
| **MSC** | 0.0004353 | 3.03555 | 0.03796 | 0.90035 | 3 |
| **MSC** | 0.0004372 | 3.17184 | 0.04145 | 0.94060 | 3 |
| **MSC** | 0.0004425 | 3.55526 | 0.05207 | 1.05368 | 3 |
| **MSC** | 0.0004460 | 3.82154 | 0.06017 | 1.13209 | 3 |
| **MSC** | 0.0004472 | 3.90834 | 0.06293 | 1.15762 | 3 |
| **MSC** | 0.0004489 | 4.04336 | 0.06735 | 1.19732 | 3 |
| **MSC** | 0.0004505 | 4.17340 | 0.07175 | 1.23552 | 3 |
| **MSC** | 0.0004505 | 4.17753 | 0.07190 | 1.23674 | 3 |
| **MSC** | 0.0004516 | 4.26385 | 0.07490 | 1.26208 | 3 |
| **MSC** | 0.0004545 | 4.51313 | 0.08391 | 1.33519 | 3 |
| **MSC** | 0.0004545 | 4.51433 | 0.08396 | 1.33554 | 3 |
| **MSC** | 0.0004596 | 4.97531 | 0.10198 | 1.47045 | 3 |
| **MSC** | 0.0004607 | 5.08193 | 0.10640 | 1.50159 | 3 |
| **MSC** | 0.0004639 | 5.41630 | 0.12086 | 1.59909 | 3 |
| **MSC** | 0.0004646 | 5.49002 | 0.12417 | 1.62056 | 3 |
| **MSC** | 0.0004660 | 5.64431 | 0.13125 | 1.66544 | 3 |
| **MSC** | 0.0004684 | 5.91774 | 0.14427 | 1.74484 | 3 |
| **FIB** | 0.4675516 | 0.18258 | 0.11576 | 0.82067 | 1 |
| **FIB** | 0.4839987 | 0.23612 | 0.19360 | 1.05981 | 1 |
| **FIB** | 0.4869078 | 0.24557 | 0.20940 | 1.10188 | 1 |
| **FIB** | 0.4873442 | 0.24698 | 0.21182 | 1.10819 | 1 |
| **FIB** | 0.4883309 | 0.25019 | 0.21735 | 1.12243 | 1 |
| **FIB** | 0.4899229 | 0.25535 | 0.22641 | 1.14541 | 1 |
| **FIB** | 0.4934438 | 0.26676 | 0.24710 | 1.19614 | 1 |
| **FIB** | 0.4995965 | 0.28668 | 0.28538 | 1.28453 | 1 |
| **FIB** | 0.5039720 | 0.30082 | 0.31424 | 1.34720 | 1 |
| **FIB** | 0.5089634 | 0.31694 | 0.34882 | 1.41848 | 1 |
| **FIB** | 0.5110941 | 0.32382 | 0.36411 | 1.44884 | 1 |
| **MSC** | 0.9996221 | -0.29026 | 0.00035 | 0.08627 | 3 |
| **MSC** | 0.9996237 | -0.36722 | 0.00056 | 0.10915 | 3 |
| **MSC** | 0.9996248 | -0.42401 | 0.00074 | 0.12602 | 3 |
| **MSC** | 0.9996274 | -0.55834 | 0.00128 | 0.16594 | 3 |
| **MSC** | 0.9996278 | -0.57760 | 0.00137 | 0.17167 | 3 |
| **MSC** | 0.9996356 | -0.96618 | 0.00385 | 0.28711 | 3 |
| **MSC** | 0.9996357 | -0.96668 | 0.00385 | 0.28726 | 3 |
| **MSC** | 0.9996357 | -0.97030 | 0.00388 | 0.28834 | 3 |
| **MSC** | 0.9996426 | -1.30521 | 0.00702 | 0.38779 | 3 |
| **MSC** | 0.9996447 | -1.40882 | 0.00818 | 0.41855 | 3 |
| **MSC** | 0.9996460 | -1.46687 | 0.00886 | 0.43578 | 3 |
| **MSC** | 0.9996505 | -1.68351 | 0.01168 | 0.50006 | 3 |
| **MSC** | 0.9996558 | -1.93499 | 0.01542 | 0.57464 | 3 |
| **MSC** | 0.9996582 | -2.04737 | 0.01727 | 0.60795 | 3 |
| **MSC** | 0.9996619 | -2.22393 | 0.02038 | 0.66027 | 3 |
| **MSC** | 0.9996683 | -2.51768 | 0.02611 | 0.74724 | 3 |
| **MSC** | 0.9996797 | -3.04183 | 0.03812 | 0.90221 | 3 |

| **DT2: Exon Arrays (N=15)** | | | | | |
|---|---|---|---|---|---|
| **Cell-types** | **p-value** | **Statistic** | **Expected** | **Std.dev** | **Residual** |
| **HSC** | 0.005409 | 8.67059 | 2.27709 | 2.50871 | 2 |
| **HSC** | 0.005799 | 9.45461 | 2.70751 | 2.67300 | 2 |
| **HSC** | 0.005918 | 9.72701 | 2.86577 | 2.72597 | 2 |
| **MSC** | 0.063132 | 1.15570 | 0.07757 | 0.70512 | 3 |
| **MSC** | 0.065092 | 1.38808 | 0.11190 | 0.84326 | 3 |
| **MSC** | 0.066603 | 1.57535 | 0.14413 | 0.95314 | 3 |
| **MSC** | 0.067632 | 1.70779 | 0.16939 | 1.02995 | 3 |
| **MSC** | 0.068559 | 1.83110 | 0.19473 | 1.10074 | 3 |
| **MSC** | 0.070686 | 2.13189 | 0.26396 | 1.27010 | 3 |
| **FIB** | 0.445009 | 0.26389 | 0.18691 | 0.55672 | 1 |
| **FIB** | 0.464887 | 0.30368 | 0.24752 | 0.63726 | 1 |
| **FIB** | 0.466542 | 0.30697 | 0.25291 | 0.64385 | 1 |
| **MSC** | 0.951724 | -0.38867 | 0.00877 | 0.23916 | 3 |
| **MSC** | 0.958045 | -0.98700 | 0.05658 | 0.60377 | 3 |
| **MSC** | 0.963863 | -1.52696 | 0.13542 | 0.92489 | 3 |

| **DT3: RNA-seq (N=29)** | | | | | |
|---|---|---|---|---|---|
| **Cell-types** | **p-value** | **Statistic** | **Expected** | **Std.dev** | **Residual** |
| **HSC** | 1.34E-07 | 4.62105 | 0.06566 | 0.88548 | 2 |
| **HSC** | 1.36E-07 | 4.85418 | 0.07245 | 0.92986 | 2 |
| **HSC** | 1.37E-07 | 5.12948 | 0.08090 | 0.98221 | 2 |
| **HSC** | 1.43E-07 | 6.07114 | 0.11333 | 1.16076 | 2 |
| **HSC** | 1.50E-07 | 7.55636 | 0.17557 | 1.44051 | 2 |
| **HSC** | 1.50E-07 | 13.88086 | 0.59244 | 2.59371 | 2 |
| **HSC** | 1.50E-07 | 13.88086 | 0.59244 | 2.59371 | 2 |
| **HSC** | 1.50E-07 | 13.88086 | 0.59244 | 2.59371 | 2 |
| **HSC** | 1.50E-07 | 13.88086 | 0.59244 | 2.59371 | 2 |
| **HSC** | 1.50E-07 | 13.88086 | 0.59244 | 2.59371 | 2 |
| **HSC** | 1.50E-07 | 13.88086 | 0.59244 | 2.59371 | 2 |
| **HSC** | 1.50E-07 | 13.88086 | 0.59244 | 2.59371 | 2 |
| **HSC** | 1.50E-07 | 13.88086 | 0.59244 | 2.59371 | 2 |
| **HSC** | 1.51E-07 | 7.82053 | 0.18806 | 1.48999 | 2 |
| **HSC** | 1.54E-07 | 8.92444 | 0.24489 | 1.69575 | 2 |
| **FIB** | 1.21E-05 | 6.29104 | 0.37993 | 1.39975 | 1 |
| **FIB** | 1.26E-05 | 6.77035 | 0.44002 | 1.50274 | 1 |
| **FIB** | 1.32E-05 | 7.28722 | 0.50977 | 1.61290 | 1 |
| **FIB** | 1.38E-05 | 7.76156 | 0.57830 | 1.71309 | 1 |
| **FIB** | 1.38E-05 | 7.81737 | 0.58665 | 1.72482 | 1 |
| **FIB** | 1.43E-05 | 8.30835 | 0.66265 | 1.82743 | 1 |
| **FIB** | 1.45E-05 | 8.45354 | 0.68601 | 1.85758 | 1 |
| **FIB** | 1.47E-05 | 8.65280 | 0.71873 | 1.89879 | 1 |
| **MSC** | 0.0015288 | 11.23015 | 0.97376 | 3.46278 | 3 |
| **MSC** | 0.0015793 | 11.75687 | 1.06724 | 3.62132 | 3 |
| **MSC** | 0.0015978 | 11.94654 | 1.10196 | 3.67828 | 3 |
| **MSC** | 0.0016116 | 12.08874 | 1.12835 | 3.72094 | 3 |
| **MSC** | 0.0016672 | 12.65170 | 1.23588 | 3.88941 | 3 |
| **MSC** | 0.0016926 | 12.90572 | 1.28601 | 3.96522 | 3 |

| **NT5E (CD73)** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **HSC** | 7.16E-13 | 43.03838 | 3.27978 | 5.61482 | 2 |
| **HSC** | 7.30E-13 | 44.15750 | 3.45257 | 5.75079 | 2 |
| **HSC** | 7.53E-13 | 46.08093 | 3.75989 | 5.98261 | 2 |
| **HSC** | 7.71E-13 | 48.16377 | 4.10747 | 6.23088 | 2 |
| **HSC** | 7.77E-13 | 49.09133 | 4.26720 | 6.34048 | 2 |
| **HSC** | 7.81E-13 | 49.74288 | 4.38122 | 6.41710 | 2 |
| **HSC** | 7.82E-13 | 49.95854 | 4.41929 | 6.44240 | 2 |
| **HSC** | 7.88E-13 | 53.81425 | 5.12776 | 6.88874 | 2 |
| **HSC** | 7.89E-13 | 53.77900 | 5.12104 | 6.88471 | 2 |
| **HSC** | 7.89E-13 | 52.76684 | 4.93009 | 6.76862 | 2 |
| **MSC** | 1.45E-06 | 0.13598 | 0.00004 | 0.02906 | 3 |
| **MSC** | 1.48E-06 | 0.46955 | 0.00050 | 0.10035 | 3 |
| **MSC** | 1.49E-06 | 0.60693 | 0.00083 | 0.12971 | 3 |
| **MSC** | 1.50E-06 | 0.75155 | 0.00127 | 0.16062 | 3 |
| **MSC** | 1.51E-06 | 0.89346 | 0.00179 | 0.19094 | 3 |
| **MSC** | 1.53E-06 | 1.17478 | 0.00310 | 0.25104 | 3 |
| **MSC** | 1.54E-06 | 1.37926 | 0.00428 | 0.29471 | 3 |
| **MSC** | 1.54E-06 | 1.39630 | 0.00438 | 0.29835 | 3 |
| **MSC** | 1.54E-06 | 1.42532 | 0.00457 | 0.30455 | 3 |
| **MSC** | 1.54E-06 | 1.42798 | 0.00458 | 0.30512 | 3 |
| **MSC** | 1.56E-06 | 1.63431 | 0.00600 | 0.34917 | 3 |
| **MSC** | 1.56E-06 | 1.65788 | 0.00618 | 0.35420 | 3 |
| **MSC** | 1.56E-06 | 1.66915 | 0.00626 | 0.35661 | 3 |
| **MSC** | 1.56E-06 | 1.72128 | 0.00666 | 0.36774 | 3 |
| **MSC** | 1.57E-06 | 1.77907 | 0.00711 | 0.38008 | 3 |
| **MSC** | 1.57E-06 | 1.82749 | 0.00751 | 0.39041 | 3 |
| **MSC** | 1.57E-06 | 1.85206 | 0.00771 | 0.39566 | 3 |
| **MSC** | 1.58E-06 | 2.00659 | 0.00905 | 0.42864 | 3 |
| **MSC** | 1.59E-06 | 2.11973 | 0.01010 | 0.45278 | 3 |
| **MSC** | 1.62E-06 | 2.64257 | 0.01570 | 0.56427 | 3 |
| **MSC** | 1.63E-06 | 2.87361 | 0.01856 | 0.61350 | 3 |
| **MSC** | 1.64E-06 | 3.08301 | 0.02136 | 0.65810 | 3 |
| **MSC** | 1.67E-06 | 3.63569 | 0.02971 | 0.77570 | 3 |
| **MSC** | 1.67E-06 | 3.63732 | 0.02974 | 0.77605 | 3 |
| **MSC** | 1.67E-06 | 3.65057 | 0.02995 | 0.77886 | 3 |
| **MSC** | 1.67E-06 | 3.67154 | 0.03030 | 0.78332 | 3 |
| **MSC** | 1.67E-06 | 3.68880 | 0.03058 | 0.78699 | 3 |
| **MSC** | 1.68E-06 | 3.78918 | 0.03227 | 0.80833 | 3 |
| **MSC** | 1.68E-06 | 3.81854 | 0.03277 | 0.81456 | 3 |
| **MSC** | 1.69E-06 | 3.94620 | 0.03500 | 0.84169 | 3 |
| **MSC** | 1.69E-06 | 3.98261 | 0.03565 | 0.84942 | 3 |
| **MSC** | 1.70E-06 | 4.28875 | 0.04134 | 0.91441 | 3 |
| **MSC** | 1.70E-06 | 4.30426 | 0.04164 | 0.91770 | 3 |
| **MSC** | 1.71E-06 | 4.45135 | 0.04453 | 0.94890 | 3 |
| **MSC** | 1.71E-06 | 4.49654 | 0.04544 | 0.95848 | 3 |
| **MSC** | 1.71E-06 | 4.56907 | 0.04692 | 0.97386 | 3 |
| **MSC** | 1.72E-06 | 4.69075 | 0.04945 | 0.99965 | 3 |
| **MSC** | 1.73E-06 | 5.05113 | 0.05734 | 1.07595 | 3 |
| **MSC** | 1.74E-06 | 5.25901 | 0.06216 | 1.11991 | 3 |
| **MSC** | 1.74E-06 | 5.27156 | 0.06246 | 1.12257 | 3 |
| **MSC** | 1.75E-06 | 5.66621 | 0.07216 | 1.20592 | 3 |
| **MSC** | 1.75E-06 | 5.68011 | 0.07251 | 1.20885 | 3 |
| **MSC** | 1.75E-06 | 5.72704 | 0.07372 | 1.21876 | 3 |
| **MSC** | 1.76E-06 | 5.77027 | 0.07484 | 1.22787 | 3 |
| **MSC** | 1.76E-06 | 5.95059 | 0.07959 | 1.26589 | 3 |
| FIB | 0.1404032 | 0.14083 | 0.00295 | 0.12784 | 1 |
| FIB | 0.1409503 | 0.15586 | 0.00362 | 0.14148 | 1 |
| FIB | 0.1472420 | 0.32648 | 0.01586 | 0.29629 | 1 |
| FIB | 0.8960651 | -0.94355 | 0.13249 | 0.85438 | 1 |
| FIB | 0.8964380 | -0.95562 | 0.13591 | 0.86526 | 1 |
| FIB | 0.8978066 | -1.00015 | 0.14887 | 0.90534 | 1 |
| FIB | 0.8989009 | -1.03599 | 0.15973 | 0.93759 | 1 |
| FIB | 0.9012212 | -1.11269 | 0.18425 | 1.00652 | 1 |
| FIB | 0.9035247 | -1.18981 | 0.21068 | 1.07573 | 1 |
| FIB | 0.9044690 | -1.22172 | 0.22213 | 1.10433 | 1 |
| FIB | 0.9083202 | -1.35367 | 0.27271 | 1.22240 | 1 |
| MSC | 0.9999986 | -0.04039 | 0.00000 | 0.00863 | 3 |
| MSC | 0.9999986 | -0.48474 | 0.00053 | 0.10360 | 3 |
| MSC | 0.9999986 | -0.82565 | 0.00153 | 0.17645 | 3 |
| MSC | 0.9999986 | -1.20233 | 0.00325 | 0.25692 | 3 |

| **DT2: Exon Arrays (N=15)** | | | | | |
|---|---|---|---|---|---|
| **Cell-types** | **p-value** | **Statistic** | **Expected** | **Std.dev** | **Residual** |
| **HSC** | 0.000914 | 10.45253 | 2.58384 | 2.52468 | 2 |
| **HSC** | 0.000918 | 11.51928 | 3.13815 | 2.69005 | 2 |
| **HSC** | 0.00092 | 11.37520 | 3.06013 | 2.66942 | 2 |
| **MSC** | 0.00814 | 0.36944 | 0.00378 | 0.15220 | 3 |
| **MSC** | 0.008272 | 0.46757 | 0.00605 | 0.19257 | 3 |
| **MSC** | 0.009009 | 1.04691 | 0.03034 | 0.42979 | 3 |
| **MSC** | 0.009117 | 1.13733 | 0.03581 | 0.46658 | 3 |
| **MSC** | 0.009741 | 1.70911 | 0.08086 | 0.69698 | 3 |
| **MSC** | 0.010046 | 2.03148 | 0.11424 | 0.82475 | 3 |
| **MSC** | 0.010305 | 2.34656 | 0.15243 | 0.94777 | 3 |
| **MSC** | 0.010486 | 2.60392 | 0.18770 | 1.04666 | 3 |
| **MSC** | 0.010568 | 2.73734 | 0.20743 | 1.09732 | 3 |
| **FIB** | 0.644873 | -0.02745 | 0.00464 | 0.08638 | 1 |
| **FIB** | 0.654873 | -0.04113 | 0.01042 | 0.12936 | 1 |
| **FIB** | 0.784508 | -0.22824 | 0.32100 | 0.69744 | 1 |

| **DT3: RNA-seq (N=29)** | | | | | |
|---|---|---|---|---|---|
| **Cell-types** | **p-value** | **Statistic** | **Expected** | **Std.dev** | **Residual** |
| **HSC** | 6.73E-07 | 20.18918 | 1.51042 | 3.86502 | 2 |
| **HSC** | 9.19E-07 | 4.06359 | 0.06119 | 0.83898 | 2 |
| **HSC** | 9.49E-07 | 4.68618 | 0.08138 | 0.96658 | 2 |
| **HSC** | 1.00E-06 | 5.95447 | 0.13139 | 1.22520 | 2 |
| **HSC** | 1.05E-06 | 7.24298 | 0.19440 | 1.48574 | 2 |
| **HSC** | 1.05E-06 | 13.23734 | 0.64933 | 2.65411 | 2 |
| **HSC** | 1.06E-06 | 7.60744 | 0.21446 | 1.55896 | 2 |
| **HSC** | 1.06E-06 | 7.65149 | 0.21695 | 1.56780 | 2 |
| **HSC** | 1.07E-06 | 8.03367 | 0.23916 | 1.64431 | 2 |
| **HSC** | 1.08E-06 | 11.87865 | 0.52287 | 2.39709 | 2 |
| **HSC** | 1.08E-06 | 8.99266 | 0.29967 | 1.83510 | 2 |
| **HSC** | 1.09E-06 | 11.21789 | 0.46632 | 2.27023 | 2 |
| **HSC** | 1.09E-06 | 11.15980 | 0.46150 | 2.25902 | 2 |
| **HSC** | 1.09E-06 | 9.91646 | 0.36440 | 2.01712 | 2 |
| **MSC** | 0.0001130 | 15.09541 | 1.20388 | 3.76663 | 3 |
| **MSC** | 0.0001356 | 18.56996 | 1.82186 | 4.59930 | 3 |
| **MSC** | 0.0001495 | 20.63291 | 2.24913 | 5.08372 | 3 |
| **MSC** | 0.0001547 | 21.39842 | 2.41912 | 5.26135 | 3 |
| **MSC** | 0.0001574 | 21.79191 | 2.50890 | 5.35218 | 3 |
| **MSC** | 0.0001727 | 24.03682 | 3.05244 | 5.86388 | 3 |
| **FIB** | 0.0003758 | 0.69417 | 0.00810 | 0.20358 | 1 |
| **FIB** | 0.0004256 | 1.34709 | 0.03051 | 0.39471 | 1 |
| **FIB** | 0.0005671 | 3.04220 | 0.15561 | 0.88684 | 1 |
| **FIB** | 0.0005813 | 3.20334 | 0.17253 | 0.93316 | 1 |
| **FIB** | 0.0007015 | 4.53054 | 0.34512 | 1.31038 | 1 |
| **FIB** | 0.0008281 | 5.90887 | 0.58705 | 1.69170 | 1 |
| **FIB** | 0.0008380 | 6.01876 | 0.60909 | 1.72154 | 1 |
| **FIB** | 0.0008488 | 6.13794 | 0.63345 | 1.75381 | 1 |
| **HSC** | 0.9999993 | -0.15096 | 0.00008 | 0.03126 | 2 |

| **TAGLN-COL4A1** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **HSC** | 1.04E-14 | 41.66120 | 2.48238 | 5.12458 | 2 |
| **HSC** | 1.11E-14 | 43.85692 | 2.74884 | 5.38234 | 2 |
| **HSC** | 1.20E-14 | 45.34097 | 2.92572 | 5.56114 | 2 |
| **HSC** | 1.28E-14 | 50.53407 | 3.63845 | 6.15548 | 2 |
| **HSC** | 1.33E-14 | 50.40108 | 3.60984 | 6.14588 | 2 |
| **HSC** | 1.34E-14 | 52.20111 | 3.87539 | 6.34797 | 2 |
| **HSC** | 1.35E-14 | 53.89513 | 4.13258 | 6.53745 | 2 |
| **HSC** | 1.38E-14 | 52.27807 | 3.88139 | 6.36018 | 2 |
| **HSC** | 1.41E-14 | 52.36937 | 3.89167 | 6.37302 | 2 |
| **HSC** | 1.43E-14 | 52.45946 | 3.90291 | 6.38516 | 2 |
| **MSC** | 1.74E-14 | 1.44009 | 0.00202 | 0.18973 | 3 |
| **MSC** | 2.86E-14 | 10.59807 | 0.09572 | 1.39763 | 3 |
| **MSC** | 3.10E-14 | 9.37825 | 0.07386 | 1.23992 | 3 |
| **MSC** | 3.11E-14 | 9.48510 | 0.07548 | 1.25405 | 3 |
| **MSC** | 3.14E-14 | 9.81427 | 0.08064 | 1.29747 | 3 |
| **MSC** | 3.18E-14 | 9.16694 | 0.07022 | 1.21285 | 3 |
| **MSC** | 3.19E-14 | 9.85744 | 0.08112 | 1.30351 | 3 |
| **MSC** | 3.23E-14 | 10.05650 | 0.08426 | 1.32990 | 3 |
| **MSC** | 3.31E-14 | 7.74725 | 0.04978 | 1.02701 | 3 |
| **MSC** | 3.40E-14 | 8.28220 | 0.05670 | 1.09797 | 3 |
| **MSC** | 3.46E-14 | 10.89191 | 0.09772 | 1.44128 | 3 |
| **MSC** | 3.49E-14 | 8.02359 | 0.05302 | 1.06440 | 3 |
| **MSC** | 3.49E-14 | 9.04594 | 0.06741 | 1.19902 | 3 |
| **MSC** | 3.53E-14 | 10.71142 | 0.09431 | 1.41810 | 3 |
| **MSC** | 3.54E-14 | 8.17015 | 0.05488 | 1.08398 | 3 |
| **MSC** | 3.57E-14 | 7.23012 | 0.04290 | 0.96016 | 3 |
| **MSC** | 3.64E-14 | 10.65569 | 0.09299 | 1.41160 | 3 |
| **MSC** | 3.64E-14 | 9.30759 | 0.07098 | 1.23441 | 3 |
| **MSC** | 3.79E-14 | 10.09903 | 0.08319 | 1.33950 | 3 |
| **MSC** | 3.91E-14 | 10.83584 | 0.09547 | 1.43717 | 3 |
| **MSC** | 3.93E-14 | 8.40251 | 0.05740 | 1.11677 | 3 |
| **MSC** | 4.03E-14 | 9.28161 | 0.06991 | 1.23329 | 3 |
| **MSC** | 4.14E-14 | 10.27896 | 0.08557 | 1.36536 | 3 |
| **MSC** | 4.26E-14 | 11.42657 | 0.10551 | 1.51718 | 3 |
| **MSC** | 4.59E-14 | 9.21304 | 0.06841 | 1.22712 | 3 |
| **MSC** | 4.88E-14 | 8.93922 | 0.06432 | 1.19219 | 3 |
| **MSC** | 4.91E-14 | 10.12958 | 0.08259 | 1.34978 | 3 |
| **MSC** | 4.96E-14 | 9.98146 | 0.08018 | 1.33045 | 3 |
| **MSC** | 5.04E-14 | 9.07887 | 0.06633 | 1.21141 | 3 |
| **MSC** | 5.08E-14 | 9.55109 | 0.07341 | 1.27409 | 3 |
| **MSC** | 5.25E-14 | 7.99468 | 0.05144 | 1.06845 | 3 |
| **MSC** | 5.41E-14 | 8.64804 | 0.06022 | 1.15575 | 3 |
| **MSC** | 5.64E-14 | 7.33542 | 0.04338 | 0.98211 | 3 |
| **MSC** | 5.79E-14 | 9.27531 | 0.06938 | 1.24042 | 3 |
| **MSC** | 7.30E-14 | 4.38363 | 0.01581 | 0.59098 | 3 |
| **MSC** | 8.19E-14 | 4.65342 | 0.01802 | 0.62849 | 3 |
| **MSC** | 8.77E-14 | 6.01073 | 0.03021 | 0.81188 | 3 |
| **MSC** | 1.03E-13 | 4.22250 | 0.01531 | 0.57280 | 3 |
| **MSC** | 1.10E-13 | 6.26755 | 0.03388 | 0.84976 | 3 |
| **MSC** | 1.38E-13 | 5.42397 | 0.02640 | 0.73884 | 3 |
| **MSC** | 1.54E-13 | 4.91657 | 0.02217 | 0.67134 | 3 |
| **MSC** | 2.46E-13 | 6.18862 | 0.03841 | 0.85092 | 3 |
| **MSC** | 2.62E-13 | 4.84760 | 0.02401 | 0.66820 | 3 |
| **MSC** | 3.27E-13 | 4.98609 | 0.02665 | 0.68987 | 3 |
| **MSC** | 5.13E-13 | 3.90490 | 0.01822 | 0.54534 | 3 |
| **MSC** | 9.69E-13 | 3.97644 | 0.02191 | 0.56181 | 3 |
| **MSC** | 4.86E-12 | 2.43649 | 0.01199 | 0.35600 | 3 |
| **MSC** | 1.48E-11 | 2.84462 | 0.02060 | 0.42478 | 3 |
| **FIB** | 1.69E-10 | 1.55371 | 0.07667 | 0.23520 | 1 |
| **FIB** | 2.91E-09 | 5.05351 | 0.27159 | 0.82134 | 1 |
| **FIB** | 2.84E-08 | 10.57682 | 0.80434 | 1.80018 | 1 |
| **FIB** | 3.13E-08 | 10.09637 | 0.70985 | 1.73470 | 1 |
| **FIB** | 5.99E-08 | 0.69583 | 0.04478 | 0.12299 | 1 |
| **FIB** | 8.54E-08 | 0.80404 | 0.06083 | 0.14214 | 1 |
| **FIB** | 2.11E-07 | 5.42414 | 0.17780 | 1.03707 | 1 |
| **FIB** | 2.16E-07 | 5.34951 | 0.17338 | 1.02418 | 1 |
| **FIB** | 3.03E-07 | 11.70735 | 0.81193 | 2.18381 | 1 |
| **FIB** | 3.88E-07 | 11.69278 | 0.81969 | 2.20037 | 1 |
| **FIB** | 4.56E-07 | 12.38921 | 0.92578 | 2.33480 | 1 |
| **MSC** | 0.391673 | 0.04447 | 0.01201 | 0.11806 | 3 |

| **DT2: Exon Arrays (N=15)** | | | | | |
|---|---|---|---|---|---|
| **Cell-types** | **p-value** | **Statistic** | **Expected** | **Std.dev** | **Residual** |
| **MSC** | 0.0002284 | 2.80435 | 0.08789 | 0.77506 | 3 |
| **MSC** | 0.0002355 | 6.34814 | 0.44167 | 1.68917 | 3 |
| **MSC** | 0.0002382 | 5.50334 | 0.32425 | 1.48242 | 3 |
| **MSC** | 0.0002387 | 3.42792 | 0.12587 | 0.94530 | 3 |
| **MSC** | 0.0002406 | 4.26962 | 0.19553 | 1.16704 | 3 |
| **MSC** | 0.0002451 | 5.99200 | 0.40639 | 1.60227 | 3 |
| **MSC** | 0.0002464 | 5.78459 | 0.37342 | 1.55286 | 3 |
| **MSC** | 0.0002493 | 3.25601 | 0.11828 | 0.90126 | 3 |
| **HSC** | 0.0011852 | 9.05753 | 1.78108 | 2.39402 | 2 |
| **HSC** | 0.0012501 | 10.03943 | 2.19741 | 2.59384 | 2 |
| **HSC** | 0.0014492 | 11.17617 | 2.70267 | 2.84507 | 2 |
| **FIB** | 0.0157632 | 1.29916 | 0.15881 | 0.53031 | 1 |
| **FIB** | 0.0159780 | 1.45173 | 0.22896 | 0.57007 | 1 |
| **FIB** | 0.0261415 | 2.40451 | 0.53683 | 0.96232 | 1 |
| **MSC** | 0.7965714 | -0.01136 | 0.13115 | 0.17182 | 3 |

| **DT3: RNA-seq (N=29)** | | | | | |
|---|---|---|---|---|---|
| **Cell-types** | **p-value** | **Statistic** | **Expected** | **Std.dev** | **Residual** |
| **HSC** | 6.19E-07 | 2.51251 | 0.06147 | 0.50542 | 2 |
| **HSC** | 8.25E-07 | 4.02870 | 0.09292 | 0.82130 | 2 |
| **HSC** | 9.14E-07 | 1.74431 | 0.01359 | 0.36272 | 2 |
| **HSC** | 1.35E-06 | 6.00797 | 0.15176 | 1.24808 | 2 |
| **HSC** | 1.54E-06 | 7.00535 | 0.20125 | 1.45841 | 2 |
| **HSC** | 1.56E-06 | 7.78670 | 0.24893 | 1.61653 | 2 |
| **HSC** | 1.65E-06 | 7.46427 | 0.22650 | 1.55630 | 2 |
| **HSC** | 1.79E-06 | 8.51511 | 0.29352 | 1.77391 | 2 |
| **HSC** | 1.90E-06 | 9.41414 | 0.35847 | 1.95910 | 2 |
| **HSC** | 1.90E-06 | 13.00565 | 0.68521 | 2.66588 | 2 |
| **HSC** | 2.05E-06 | 11.51865 | 0.53902 | 2.38361 | 2 |
| **HSC** | 2.08E-06 | 16.69020 | 1.19737 | 3.36548 | 2 |
| **HSC** | 2.38E-06 | 10.23305 | 0.43437 | 2.14187 | 2 |
| **HSC** | 3.02E-06 | 14.65401 | 1.04656 | 3.00709 | 2 |
| **HSC** | 1.99E-05 | 0.92428 | 0.06421 | 0.20931 | 2 |
| **MSC** | 3.85E-05 | 19.25468 | 1.75458 | 4.42632 | 3 |
| **MSC** | 4.34E-05 | 23.98714 | 2.72977 | 5.41652 | 3 |
| **MSC** | 4.43E-05 | 24.37896 | 2.81790 | 5.50073 | 3 |
| **MSC** | 4.45E-05 | 22.67340 | 2.43212 | 5.16532 | 3 |
| **MSC** | 4.48E-05 | 24.38824 | 2.81696 | 5.50721 | 3 |
| **MSC** | 5.57E-05 | 26.07202 | 3.24805 | 5.90630 | 3 |
| **FIB** | 0.0010436 | 0.53830 | 0.00776 | 0.17239 | 1 |
| **FIB** | 0.0016416 | 2.06068 | 0.09545 | 0.66847 | 1 |
| **FIB** | 0.0016997 | 1.86355 | 0.07646 | 0.61012 | 1 |
| **FIB** | 0.0019906 | 2.86760 | 0.18130 | 0.93286 | 1 |
| **FIB** | 0.0020169 | 3.06416 | 0.20790 | 0.99331 | 1 |
| **FIB** | 0.0021783 | 3.66790 | 0.30014 | 1.18120 | 1 |
| **FIB** | 0.0026153 | 4.58025 | 0.46185 | 1.47482 | 1 |
| **FIB** | 0.0030341 | 3.18046 | 0.26177 | 1.06363 | 1 |

| **ENG-NT5E** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **HSC** | 4.55E-13 | 43.88866 | 3.40458 | 5.66738 | 2 |
| **HSC** | 4.87E-13 | 54.67000 | 5.27848 | 6.92322 | 2 |
| **HSC** | 4.90E-13 | 49.59185 | 4.33875 | 6.34397 | 2 |
| **HSC** | 4.94E-13 | 50.03689 | 4.41557 | 6.39662 | 2 |
| **HSC** | 4.98E-13 | 52.96275 | 4.94691 | 6.73345 | 2 |
| **HSC** | 5.00E-13 | 53.84184 | 5.11307 | 6.83389 | 2 |
| **HSC** | 5.05E-13 | 49.62410 | 4.34760 | 6.35097 | 2 |
| **HSC** | 5.68E-13 | 43.66782 | 3.47422 | 5.65069 | 2 |
| **HSC** | 5.88E-13 | 41.32078 | 3.16093 | 5.36843 | 2 |
| **HSC** | 6.05E-13 | 45.31797 | 3.77418 | 5.84767 | 2 |
| **MSC** | 7.41E-08 | 3.81260 | 0.06118 | 0.71391 | 3 |
| **MSC** | 7.51E-08 | 4.23690 | 0.08985 | 0.78956 | 3 |
| **MSC** | 7.82E-08 | 2.01031 | 0.01326 | 0.38076 | 3 |
| **MSC** | 1.06E-07 | 5.47345 | 0.08237 | 1.03918 | 3 |
| **MSC** | 1.08E-07 | 5.50008 | 0.08261 | 1.04473 | 3 |
| **MSC** | 1.19E-07 | 6.58670 | 0.11240 | 1.25317 | 3 |
| **MSC** | 1.40E-07 | 7.20613 | 0.12586 | 1.37860 | 3 |
| **MSC** | 1.49E-07 | 7.77148 | 0.14327 | 1.48851 | 3 |
| **MSC** | 1.50E-07 | 3.16306 | 0.08066 | 0.60156 | 3 |
| **MSC** | 1.59E-07 | 7.51420 | 0.13148 | 1.44394 | 3 |
| **MSC** | 1.79E-07 | 5.10598 | 0.05845 | 0.99174 | 3 |
| **MSC** | 1.81E-07 | 1.72290 | 0.00649 | 0.33732 | 3 |
| **MSC** | 2.11E-07 | 2.02350 | 0.00872 | 0.39827 | 3 |
| **MSC** | 2.17E-07 | 1.67870 | 0.00596 | 0.33099 | 3 |
| **MSC** | 2.21E-07 | 1.70769 | 0.00616 | 0.33693 | 3 |
| **MSC** | 2.25E-07 | 1.98309 | 0.00829 | 0.39134 | 3 |
| **MSC** | 2.37E-07 | 1.68712 | 0.00596 | 0.33381 | 3 |
| **MSC** | 2.56E-07 | 7.10999 | 0.10653 | 1.39453 | 3 |
| **MSC** | 3.13E-07 | 5.10084 | 0.05363 | 1.01286 | 3 |
| **MSC** | 3.47E-07 | 2.35554 | 0.01135 | 0.47231 | 3 |
| **MSC** | 3.65E-07 | 2.03678 | 0.00848 | 0.40951 | 3 |
| **MSC** | 3.70E-07 | 6.40163 | 0.08392 | 1.27615 | 3 |
| **MSC** | 3.80E-07 | 3.39460 | 0.02357 | 0.68169 | 3 |
| **MSC** | 4.02E-07 | 4.18261 | 0.03578 | 0.84041 | 3 |
| **MSC** | 4.38E-07 | 5.08779 | 0.05298 | 1.02380 | 3 |
| **MSC** | 4.53E-07 | 5.69684 | 0.06646 | 1.14652 | 3 |
| **MSC** | 4.72E-07 | 3.88372 | 0.03097 | 0.78577 | 3 |
| **MSC** | 5.18E-07 | 3.84367 | 0.03049 | 0.78066 | 3 |
| **MSC** | 6.07E-07 | 4.44213 | 0.04116 | 0.90677 | 3 |
| **MSC** | 7.09E-07 | 4.33223 | 0.03979 | 0.89007 | 3 |
| **MSC** | 1.01E-06 | 2.75842 | 0.01710 | 0.57692 | 3 |
| **MSC** | 1.06E-06 | 5.46812 | 0.06694 | 1.13908 | 3 |
| **MSC** | 1.60E-06 | 3.59957 | 0.03175 | 0.76610 | 3 |
| **MSC** | 4.80E-06 | 1.43268 | 0.00684 | 0.32214 | 3 |
| **MSC** | 4.83E-06 | 1.31435 | 0.00578 | 0.29575 | 3 |
| **MSC** | 4.90E-06 | 3.06695 | 0.03087 | 0.68668 | 3 |
| **MSC** | 3.03E-05 | 1.79002 | 0.01855 | 0.44172 | 3 |
| **MSC** | 5.11E-05 | 2.91666 | 0.05702 | 0.73603 | 3 |
| **MSC** | 8.18E-05 | 3.13769 | 0.07641 | 0.81214 | 3 |
| **MSC** | 0.000173 | 2.12850 | 0.04509 | 0.58225 | 3 |
| **MSC** | 0.0001959 | 0.42251 | 0.00194 | 0.11862 | 3 |
| **MSC** | 0.0080054 | 0.73633 | 0.02196 | 0.29658 | 3 |
| **FIB** | 0.0089849 | 0.58509 | 0.02453 | 0.23690 | 1 |
| **FIB** | 0.0097707 | 0.62232 | 0.02744 | 0.25476 | 1 |
| **FIB** | 0.0233881 | 0.85290 | 0.04677 | 0.40543 | 1 |
| **FIB** | 0.7976914 | -0.44565 | 0.14180 | 0.70488 | 1 |
| **FIB** | 0.8218549 | -0.52405 | 0.13167 | 0.71084 | 1 |
| **FIB** | 0.8350825 | -0.66269 | 0.20921 | 0.89477 | 1 |
| **FIB** | 0.8368003 | -0.61710 | 0.15574 | 0.78749 | 1 |
| **MSC** | 0.8418830 | -0.14251 | 0.01226 | 0.15442 | 3 |
| **FIB** | 0.8503312 | -0.79592 | 0.26683 | 1.02399 | 1 |
| **FIB** | 0.8503386 | -0.66036 | 0.14139 | 0.77248 | 1 |
| **FIB** | 0.9092539 | -1.14765 | 0.20164 | 1.00982 | 1 |
| **FIB** | 0.9326749 | -1.27612 | 0.17456 | 0.96970 | 1 |
| **MSC** | 0.9999378 | -0.74370 | 0.00436 | 0.19495 | 3 |
| **MSC** | 0.9999561 | -0.70044 | 0.01530 | 0.18249 | 3 |
| **MSC** | 0.9999983 | -0.65252 | 0.00708 | 0.14186 | 3 |
| **MSC** | 0.9999998 | -1.11289 | 0.01356 | 0.22317 | 3 |
| **MSC** | 0.9999999 | -0.67310 | 0.00112 | 0.12981 | 3 |
| **MSC** | 1.0000000 | -1.69186 | 0.01180 | 0.31905 | 3 |

| **DT2: Exon Arrays (N=15)** | | | | | |
|---|---|---|---|---|---|
| **Cell-types** | **p-value** | **Statistic** | **Expected** | **Std.dev** | **Residual** |
| **HSC** | 0.0007438 | 10.50290 | 2.59180 | 2.49005 | 2 |
| **HSC** | 0.0007502 | 11.50277 | 3.11123 | 2.64333 | 2 |
| **HSC** | 0.0007699 | 11.27148 | 2.99455 | 2.61342 | 2 |
| **MSC** | 0.0017550 | 1.09642 | 0.11730 | 0.33541 | 3 |
| **MSC** | 0.0038641 | 1.45132 | 0.07352 | 0.51725 | 3 |
| **MSC** | 0.0047289 | 0.54087 | 0.00784 | 0.20540 | 3 |
| **MSC** | 0.0066042 | 1.84683 | 0.08817 | 0.70968 | 3 |
| **MSC** | 0.0069645 | 2.17403 | 0.12212 | 0.83442 | 3 |
| **MSC** | 0.0075464 | 1.13506 | 0.03451 | 0.45287 | 3 |
| **MSC** | 0.0086896 | 2.73767 | 0.20034 | 1.06674 | 3 |
| **MSC** | 0.0107710 | 2.15998 | 0.14258 | 0.87776 | 3 |
| **MSC** | 0.0118162 | 2.33927 | 0.17706 | 0.95544 | 3 |
| **FIB** | 0.4047882 | 0.02674 | 0.01159 | 0.06286 | 1 |
| **FIB** | 0.5121777 | 0.01388 | 0.01697 | 0.10111 | 1 |
| **FIB** | 0.7777400 | -0.18800 | 0.30150 | 0.64022 | 1 |

| **DT3: RNA-seq (N=29)** | | | | | |
|---|---|---|---|---|---|
| **Cell-types** | **p-value** | **Statistic** | **Expected** | **Std.dev** | **Residual** |
| **HSC** | 5.04E-07 | 5.02824 | 0.11274 | 1.00519 | 2 |
| **HSC** | 5.38E-07 | 17.31212 | 1.25282 | 3.29260 | 2 |
| **HSC** | 5.48E-07 | 9.30424 | 0.36836 | 1.83348 | 2 |
| **HSC** | 5.48E-07 | 8.66084 | 0.39886 | 1.69524 | 2 |
| **HSC** | 5.92E-07 | 7.76352 | 0.24216 | 1.54817 | 2 |
| **HSC** | 6.36E-07 | 5.41466 | 0.11716 | 1.09363 | 2 |
| **HSC** | 6.43E-07 | 9.26585 | 0.34182 | 1.84305 | 2 |
| **HSC** | 6.57E-07 | 7.01135 | 0.19604 | 1.40881 | 2 |
| **HSC** | 6.74E-07 | 6.90092 | 0.19047 | 1.38863 | 2 |
| **HSC** | 7.40E-07 | 11.65714 | 0.55300 | 2.30665 | 2 |
| **HSC** | 7.45E-07 | 9.90261 | 0.39679 | 1.97517 | 2 |
| **HSC** | 7.67E-07 | 10.43160 | 0.44385 | 2.07789 | 2 |
| **HSC** | 7.68E-07 | 9.86700 | 0.39658 | 1.97032 | 2 |
| **HSC** | 7.69E-07 | 3.42111 | 0.05015 | 0.70138 | 2 |
| **MSC** | 8.73E-05 | 13.71405 | 1.07656 | 3.36704 | 3 |
| **MSC** | 0.0001194 | 20.55487 | 2.50056 | 4.91403 | 3 |
| **MSC** | 0.0001204 | 20.83704 | 2.52351 | 4.98759 | 3 |
| **MSC** | 0.0001299 | 15.25586 | 1.48096 | 3.77155 | 3 |
| **HSC** | 0.0001470 | 0.60618 | 0.01664 | 0.16283 | 2 |
| **MSC** | 0.0001482 | 23.61335 | 3.67223 | 5.51088 | 3 |
| **MSC** | 0.0002316 | 18.33066 | 2.54496 | 4.50863 | 3 |
| **FIB** | 0.0003914 | 1.76771 | 0.08656 | 0.50052 | 1 |
| **FIB** | 0.0004318 | 3.05857 | 0.16856 | 0.86746 | 1 |
| **FIB** | 0.0004651 | 3.44689 | 0.25612 | 0.96374 | 1 |
| **FIB** | 0.0005360 | 4.18956 | 0.31572 | 1.18433 | 1 |
| **FIB** | 0.0007309 | 5.26168 | 0.52487 | 1.48856 | 1 |
| **FIB** | 0.0008517 | 4.75821 | 0.47697 | 1.36450 | 1 |
| **FIB** | 0.0009549 | 4.71283 | 0.49881 | 1.35765 | 1 |
| **FIB** | 0.0012782 | 1.75807 | 0.20074 | 0.51626 | 1 |

| **TAGLN-COL4A1-COL4A2-SCUBE3** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **MSC** | 2.27E-14 | 12.30409 | 0.13975 | 1.61230 | 3 |
| **MSC** | 2.38E-14 | 11.54859 | 0.11688 | 1.51644 | 3 |
| **MSC** | 2.42E-14 | 11.23501 | 0.10886 | 1.47631 | 3 |
| **MSC** | 2.42E-14 | 11.33375 | 0.11124 | 1.48910 | 3 |
| **MSC** | 2.43E-14 | 11.59290 | 0.12022 | 1.52242 | 3 |
| **MSC** | 2.45E-14 | 9.33193 | 0.07828 | 1.22814 | 3 |
| **MSC** | 2.48E-14 | 8.05032 | 0.05348 | 1.06161 | 3 |
| **HSC** | 2.52E-14 | 39.58970 | 2.31092 | 4.95023 | 2 |
| **MSC** | 2.53E-14 | 11.94392 | 0.11884 | 1.57034 | 3 |
| **MSC** | 2.58E-14 | 9.82908 | 0.08891 | 1.29389 | 3 |
| **MSC** | 2.63E-14 | 11.07100 | 0.11276 | 1.45614 | 3 |
| **MSC** | 2.65E-14 | 10.15867 | 0.08831 | 1.33833 | 3 |
| **MSC** | 2.70E-14 | 11.80192 | 0.11380 | 1.55384 | 3 |
| **MSC** | 2.72E-14 | 10.86400 | 0.09829 | 1.43141 | 3 |
| **MSC** | 2.72E-14 | 9.84715 | 0.08210 | 1.29838 | 3 |
| **MSC** | 2.73E-14 | 10.40465 | 0.09884 | 1.37036 | 3 |
| **HSC** | 2.76E-14 | 49.10474 | 3.62802 | 6.04838 | 2 |
| **MSC** | 2.77E-14 | 9.51706 | 0.07630 | 1.25568 | 3 |
| **HSC** | 2.84E-14 | 42.89300 | 2.70516 | 5.34760 | 2 |
| **HSC** | 2.87E-14 | 49.10682 | 3.63058 | 6.05230 | 2 |
| **MSC** | 2.88E-14 | 9.10394 | 0.06778 | 1.20268 | 3 |
| **HSC** | 2.89E-14 | 49.48528 | 3.69630 | 6.09477 | 2 |
| **HSC** | 2.91E-14 | 41.56784 | 2.55071 | 5.19404 | 2 |
| **MSC** | 2.96E-14 | 11.75352 | 0.11089 | 1.55031 | 3 |
| **MSC** | 3.02E-14 | 9.69198 | 0.07481 | 1.28106 | 3 |
| **MSC** | 3.07E-14 | 11.47174 | 0.10450 | 1.51460 | 3 |
| MSC | 3.13E-14 | 10.38799 | 0.08582 | 1.37315 | 3 |
| MSC | 3.17E-14 | 10.30812 | 0.08440 | 1.36304 | 3 |
| **MSC** | 3.38E-14 | 6.22480 | 0.04463 | 0.82487 | 3 |
| **HSC** | 3.46E-14 | 51.80703 | 3.94071 | 6.39122 | 2 |
| **MSC** | 3.47E-14 | 4.31555 | 0.01647 | 0.57406 | 3 |
| **HSC** | 3.69E-14 | 48.65927 | 3.48220 | 6.03885 | 2 |
| **HSC** | 3.87E-14 | 50.62570 | 3.75316 | 6.27075 | 2 |
| **MSC** | 3.89E-14 | 8.46854 | 0.05700 | 1.12546 | 3 |
| **HSC** | 4.18E-14 | 48.56160 | 3.48308 | 6.03912 | 2 |
| **MSC** | 4.47E-14 | 5.40572 | 0.03226 | 0.72072 | 3 |
| **MSC** | 4.51E-14 | 10.92608 | 0.11057 | 1.45085 | 3 |
| **MSC** | 4.62E-14 | 9.88503 | 0.08905 | 1.31466 | 3 |
| **MSC** | 4.65E-14 | 8.66346 | 0.06414 | 1.15418 | 3 |
| **MSC** | 4.90E-14 | 9.59988 | 0.08473 | 1.27829 | 3 |
| **MSC** | 5.69E-14 | 3.68802 | 0.01216 | 0.49515 | 3 |
| **MSC** | 5.74E-14 | 8.90191 | 0.07310 | 1.18944 | 3 |
| **MSC** | 7.17E-14 | 9.82853 | 0.09248 | 1.31690 | 3 |
| **MSC** | 7.23E-14 | 4.40314 | 0.01797 | 0.59324 | 3 |
| **MSC** | 8.00E-14 | 4.62920 | 0.02038 | 0.62462 | 3 |
| **MSC** | 8.03E-14 | 8.49515 | 0.07519 | 1.14121 | 3 |
| **MSC** | 8.93E-14 | 3.81304 | 0.01361 | 0.51596 | 3 |
| **MSC** | 9.21E-14 | 7.10408 | 0.05464 | 0.95783 | 3 |
| **MSC** | 9.74E-14 | 7.02552 | 0.04915 | 0.94887 | 3 |
| **MSC** | 1.07E-13 | 6.63427 | 0.04426 | 0.89790 | 3 |
| **MSC** | 1.38E-13 | 4.97983 | 0.02834 | 0.67778 | 3 |
| **MSC** | 3.93E-13 | 3.81471 | 0.01931 | 0.52982 | 3 |
| **MSC** | 5.84E-13 | 3.44217 | 0.01935 | 0.48147 | 3 |
| **MSC** | 5.89E-12 | 4.54102 | 0.05431 | 0.66148 | 3 |
| **MSC** | 5.92E-12 | 2.27136 | 0.01421 | 0.33280 | 3 |
| **MSC** | 1.10E-10 | 2.26274 | 0.01967 | 0.35343 | 3 |
| **FIB** | 3.04E-10 | 2.51814 | 0.09636 | 0.39133 | 1 |
| **FIB** | 4.29E-10 | 2.05215 | 0.09421 | 0.31920 | 1 |
| **FIB** | 9.53E-10 | 1.59385 | 0.07598 | 0.25274 | 1 |
| **FIB** | 2.11E-09 | 6.79289 | 0.42528 | 1.08386 | 1 |
| **FIB** | 1.32E-08 | 11.35436 | 0.79705 | 1.89761 | 1 |
| **FIB** | 1.85E-08 | 13.51943 | 1.13341 | 2.25025 | 1 |
| **FIB** | 4.82E-08 | 5.70154 | 0.17979 | 1.03534 | 1 |
| **FIB** | 5.80E-08 | 5.58777 | 0.17300 | 1.02171 | 1 |
| **FIB** | 1.08E-07 | 12.54060 | 0.86007 | 2.25309 | 1 |
| **FIB** | 1.24E-07 | 12.60890 | 0.87452 | 2.27420 | 1 |
| **MSC** | 1.50E-07 | 0.58369 | 0.00640 | 0.11267 | 3 |
| **FIB** | 1.50E-07 | 13.01158 | 0.93935 | 2.35627 | 1 |
| **MSC** | 0.000548 | 0.40497 | 0.01377 | 0.11982 | 3 |
| **MSC** | 1 | -1.90307 | 0.01945 | 0.27682 | 3 |

| **DT2: Exon Arrays (N=15)** | | | | | |
|---|---|---|---|---|---|
| **Cell-types** | **p-value** | **Statistic** | **Expected** | **Std.dev** | **Residual** |
| **MSC** | 0.000159 | 7.43830 | 0.61949 | 1.89378 | 3 |
| **MSC** | 0.000191 | 4.43505 | 0.20795 | 1.18988 | 3 |
| **MSC** | 0.000196 | 6.31501 | 0.42334 | 1.66183 | 3 |
| **MSC** | 0.000198 | 3.69612 | 0.16999 | 0.99538 | 3 |
| **MSC** | 0.0002 | 3.80066 | 0.15680 | 1.02915 | 3 |
| **MSC** | 0.0002 | 4.85791 | 0.28426 | 1.29206 | 3 |
| **MSC** | 0.000254 | 4.65575 | 0.29516 | 1.25421 | 3 |
| **MSC** | 0.000294 | 4.46552 | 0.34033 | 1.20005 | 3 |
| **HSC** | 0.00181 | 7.74987 | 1.43039 | 2.17201 | 2 |
| **HSC** | 0.0021 | 8.92889 | 1.89463 | 2.45712 | 2 |
| **HSC** | 0.00233 | 10.01012 | 2.39350 | 2.69175 | 2 |
| **FIB** | 0.009758 | 2.02058 | 0.32097 | 0.72773 | 1 |
| **FIB** | 0.010714 | 2.33352 | 0.44515 | 0.82091 | 1 |
| **FIB** | 0.014099 | 2.59755 | 0.51545 | 0.94877 | 1 |
| **MSC** | 0.999849 | -0.96738 | 0.08021 | 0.28984 | 3 |

| **DT3: RNA-seq (N=29)** | | | | | |
|---|---|---|---|---|---|
| **Cell-types** | **p-value** | **Statistic** | **Expected** | **Std.dev** | **Residual** |
| **HSC** | 5.86E-07 | 0.81117 | 0.00912 | 0.16502 | 2 |
| **HSC** | 1.76E-06 | 6.85368 | 0.21470 | 1.43151 | 2 |
| **HSC** | 1.80E-06 | 8.29917 | 0.31848 | 1.72250 | 2 |
| **HSC** | 1.83E-06 | 12.69874 | 0.73515 | 2.58397 | 2 |
| **HSC** | 1.93E-06 | 5.81152 | 0.15542 | 1.22447 | 2 |
| **HSC** | 2.05E-06 | 9.47334 | 0.40857 | 1.96777 | 2 |
| **HSC** | 2.05E-06 | 6.43623 | 0.18702 | 1.35674 | 2 |
| **HSC** | 2.11E-06 | 7.50885 | 0.25611 | 1.57665 | 2 |
| **HSC** | 2.13E-06 | 14.66416 | 1.07490 | 2.95500 | 2 |
| **HSC** | 2.24E-06 | 11.18359 | 0.56357 | 2.31505 | 2 |
| **HSC** | 2.26E-06 | 12.52296 | 0.77828 | 2.56085 | 2 |
| **HSC** | 2.36E-06 | 8.38107 | 0.32432 | 1.76031 | 2 |
| **HSC** | 3.85E-06 | 3.07829 | 0.07777 | 0.67073 | 2 |
| **MSC** | 1.27E-05 | 21.75210 | 2.59865 | 4.54876 | 3 |
| **HSC** | 1.65E-05 | 2.10303 | 0.07189 | 0.48920 | 2 |
| **MSC** | 2.18E-05 | 22.05053 | 2.44159 | 4.79680 | 3 |
| **MSC** | 2.22E-05 | 22.71007 | 2.58648 | 4.92780 | 3 |
| **MSC** | 2.27E-05 | 22.55500 | 2.55019 | 4.90485 | 3 |
| **MSC** | 2.40E-05 | 22.02174 | 2.42295 | 4.82073 | 3 |
| **FIB** | 2.99E-05 | 0.98537 | 0.09888 | 0.22089 | 1 |
| **MSC** | 3.61E-05 | 24.53639 | 3.02705 | 5.41911 | 3 |
| **FIB** | 9.02E-05 | 2.04586 | 0.17839 | 0.49864 | 1 |
| **FIB** | 0.0001293 | 2.05520 | 0.15866 | 0.51907 | 1 |
| **FIB** | 0.0006800 | 3.51158 | 0.30402 | 1.00141 | 1 |
| **FIB** | 0.0013731 | 2.83586 | 0.18684 | 0.88454 | 1 |
| **FIB** | 0.0020858 | 3.51199 | 0.31528 | 1.11583 | 1 |
| **FIB** | 0.0023773 | 2.63791 | 0.17272 | 0.87319 | 1 |
| **FIB** | 0.0024532 | 4.21807 | 0.42424 | 1.34862 | 1 |
| **HSC** | 0.3264260 | 0.14492 | 0.06202 | 0.18431 | 2 |

| **ENG-THYI-NT5E** | | | | | |
|---|---|---|---|---|---|
| **DT1: Microarrays (Metanalysis) (N=70)** | | | | | |
| **Cell-types** | **p-value** | **Statistic** | **Expected** | **Std.dev** | **Residual** |
| **HSC** | 1.83E-12 | 40.16181 | 3.13275 | 5.32832 | 2 |
| **HSC** | 1.92E-12 | 41.65742 | 3.39148 | 5.51138 | 2 |
| **HSC** | 1.94E-12 | 38.59309 | 2.92251 | 5.13869 | 2 |
| **HSC** | 2.07E-12 | 47.01016 | 4.19403 | 6.17637 | 2 |
| **HSC** | 2.29E-12 | 50.72652 | 4.89159 | 6.62566 | 2 |
| **HSC** | 2.31E-12 | 52.46237 | 5.23828 | 6.82724 | 2 |
| **HSC** | 2.37E-12 | 47.96780 | 4.38300 | 6.30449 | 2 |
| **HSC** | 2.43E-12 | 43.28499 | 3.58613 | 5.74563 | 2 |
| **HSC** | 2.46E-12 | 52.39594 | 5.23264 | 6.82722 | 2 |
| **HSC** | 2.48E-12 | 48.46842 | 4.48211 | 6.36865 | 2 |
| **MSC** | 6.22E-09 | 2.20563 | 0.04812 | 0.37894 | 3 |
| **MSC** | 2.57E-08 | 1.77486 | 0.02610 | 0.32110 | 3 |
| **MSC** | 4.93E-08 | 0.48865 | 0.00799 | 0.09020 | 3 |
| **MSC** | 1.33E-07 | 1.36724 | 0.00758 | 0.26422 | 3 |
| **MSC** | 1.53E-07 | 1.31355 | 0.01352 | 0.25392 | 3 |
| **MSC** | 6.65E-07 | 2.86264 | 0.02338 | 0.58721 | 3 |
| **MSC** | 8.25E-07 | 3.39330 | 0.04334 | 0.69906 | 3 |
| **MSC** | 8.84E-07 | 3.98644 | 0.04398 | 0.82510 | 3 |
| **MSC** | 1.25E-06 | 6.27236 | 0.11085 | 1.30869 | 3 |
| **MSC** | 1.54E-06 | 3.11470 | 0.02685 | 0.66184 | 3 |
| **MSC** | 1.63E-06 | 4.95349 | 0.06203 | 1.05113 | 3 |
| **MSC** | 1.70E-06 | 6.08157 | 0.10506 | 1.28659 | 3 |
| **MSC** | 1.84E-06 | 1.39245 | 0.00496 | 0.29975 | 3 |
| **MSC** | 2.10E-06 | 0.42230 | 0.00966 | 0.08968 | 3 |
| **MSC** | 2.23E-06 | 5.84942 | 0.10227 | 1.25251 | 3 |
| **MSC** | 2.57E-06 | 4.14255 | 0.04279 | 0.89922 | 3 |
| **MSC** | 2.62E-06 | 6.06585 | 0.09416 | 1.31094 | 3 |
| **MSC** | 2.93E-06 | 6.76827 | 0.12770 | 1.46539 | 3 |
| **MSC** | 3.44E-06 | 4.68014 | 0.06005 | 1.02730 | 3 |
| **MSC** | 3.71E-06 | 5.31349 | 0.08949 | 1.16569 | 3 |
| **MSC** | 3.89E-06 | 5.33424 | 0.09091 | 1.17276 | 3 |
| **MSC** | 5.07E-06 | 3.92580 | 0.03962 | 0.88039 | 3 |
| **MSC** | 5.30E-06 | 2.90113 | 0.02438 | 0.65316 | 3 |
| **MSC** | 5.93E-06 | 1.08127 | 0.02918 | 0.24020 | 3 |
| **MSC** | 5.94E-06 | 4.73732 | 0.10457 | 1.05780 | 3 |
| **MSC** | 6.02E-06 | 3.55633 | 0.03314 | 0.80496 | 3 |
| **MSC** | 8.16E-06 | 2.31556 | 0.02060 | 0.53246 | 3 |
| **MSC** | 9.25E-06 | 4.56111 | 0.09072 | 1.04390 | 3 |
| **MSC** | 9.33E-06 | 3.99044 | 0.09613 | 0.90983 | 3 |
| **MSC** | 1.36E-05 | 3.79273 | 0.04504 | 0.89336 | 3 |
| **MSC** | 2.04E-05 | 1.41316 | 0.00719 | 0.34268 | 3 |
| **MSC** | 2.19E-05 | 2.66281 | 0.02701 | 0.64500 | 3 |
| **MSC** | 2.65E-05 | 1.41222 | 0.00742 | 0.34755 | 3 |
| **MSC** | 2.86E-05 | 2.79404 | 0.03198 | 0.68644 | 3 |
| **MSC** | 2.94E-05 | 2.79795 | 0.03103 | 0.68875 | 3 |
| **MSC** | 5.07E-05 | 1.91637 | 0.03676 | 0.48351 | 3 |
| **MSC** | 5.35E-05 | 1.87656 | 0.01801 | 0.47972 | 3 |
| **MSC** | 7.46E-05 | 2.35673 | 0.05054 | 0.60810 | 3 |
| **MSC** | 0.0002072 | 1.26848 | 0.02918 | 0.35101 | 3 |
| **MSC** | 0.0002503 | 1.40006 | 0.01599 | 0.39767 | 3 |
| **MSC** | 0.0009428 | 0.30976 | 0.01121 | 0.09607 | 3 |
| **FIB** | 0.0044615 | 0.64043 | 0.11065 | 0.20260 | 1 |
| **MSC** | 0.0533591 | 0.26914 | 0.01464 | 0.15777 | 3 |
| **FIB** | 0.0654463 | 0.48886 | 0.09300 | 0.26206 | 1 |
| **FIB** | 0.1105504 | 0.47190 | 0.10367 | 0.30094 | 1 |
| **FIB** | 0.6746823 | -0.20324 | 0.16022 | 0.80255 | 1 |
| **FIB** | 0.6793539 | -0.22503 | 0.20888 | 0.93134 | 1 |
| **FIB** | 0.7006566 | -0.29072 | 0.21210 | 0.95540 | 1 |
| **FIB** | 0.7055726 | -0.31043 | 0.22058 | 0.98244 | 1 |
| **MSC** | 0.7239128 | -0.04063 | 0.00504 | 0.07681 | 3 |
| **FIB** | 0.7382420 | -0.36215 | 0.17598 | 0.84355 | 1 |
| **FIB** | 0.7448792 | -0.42560 | 0.24641 | 1.02059 | 1 |
| **FIB** | 0.7988896 | -0.65064 | 0.20723 | 1.02412 | 1 |
| **FIB** | 0.8118629 | -0.72045 | 0.21396 | 1.05609 | 1 |
| **MSC** | 0.9998408 | -0.59276 | 0.01081 | 0.16766 | 3 |
| **MSC** | 0.9999914 | -0.77878 | 0.00210 | 0.18172 | 3 |
| **MSC** | 0.9999925 | -0.64992 | 0.01032 | 0.15248 | 3 |
| **MSC** | 0.9999954 | -0.76422 | 0.00593 | 0.17366 | 3 |
| **MSC** | 0.9999969 | -1.23876 | 0.01189 | 0.27659 | 3 |
| **MSC** | 0.9999993 | -1.43384 | 0.00897 | 0.29859 | 3 |

| **DT2: Exon Arrays (N=15)** | | | | | |
|---|---|---|---|---|---|
| **Cell-types** | **p-value** | **Statistic** | **Expected** | **Std.dev** | **Residual** |
| **HSC** | 0.0007485 | 10.88224 | 2.98891 | 2.48587 | 2 |
| **HSC** | 0.0007552 | 9.94776 | 2.49645 | 2.34857 | 2 |
| **HSC** | 0.0007650 | 10.80355 | 2.95553 | 2.47653 | 2 |
| **MSC** | 0.0081205 | 1.79048 | 0.14230 | 0.68576 | 3 |
| **MSC** | 0.0095342 | 1.36176 | 0.07474 | 0.54902 | 3 |
| **MSC** | 0.0119806 | 1.33151 | 0.14055 | 0.52750 | 3 |
| **MSC** | 0.0121815 | 1.99265 | 0.12879 | 0.82788 | 3 |
| **MSC** | 0.0135044 | 1.80471 | 0.11278 | 0.76510 | 3 |
| **MSC** | 0.0135815 | 1.04294 | 0.14041 | 0.40854 | 3 |
| **MSC** | 0.0173043 | 1.41014 | 0.16173 | 0.59085 | 3 |
| **MSC** | 0.0196787 | 1.34594 | 0.08305 | 0.61292 | 3 |
| **MSC** | 0.0231654 | 0.79755 | 0.03937 | 0.38055 | 3 |
| **FIB** | 0.4504793 | 0.11064 | 0.08307 | 0.22156 | 1 |
| **FIB** | 0.4771184 | 0.10268 | 0.08845 | 0.24791 | 1 |
| **FIB** | 0.7068391 | -0.05109 | 0.26679 | 0.58414 | 1 |

| **DT3: RNA-seq (N=29)** | | | | | |
|---|---|---|---|---|---|
| **Cell-types** | **p-value** | **Statistic** | **Expected** | **Std.dev** | **Residual** |
| **HSC** | 8.36E-08 | 12.54101 | 0.53472 | 2.29457 | 2 |
| **HSC** | 8.43E-08 | 12.32169 | 0.51636 | 2.25683 | 2 |
| **HSC** | 8.53E-08 | 12.10308 | 0.50540 | 2.21802 | 2 |
| **HSC** | 8.79E-08 | 7.56108 | 0.19528 | 1.41017 | 2 |
| **HSC** | 8.81E-08 | 11.50460 | 0.45638 | 2.11538 | 2 |
| **HSC** | 9.00E-08 | 11.16952 | 0.43630 | 2.05658 | 2 |
| **HSC** | 9.21E-08 | 8.22041 | 0.24015 | 1.53036 | 2 |
| **HSC** | 9.28E-08 | 5.69213 | 0.12046 | 1.06877 | 2 |
| **HSC** | 9.45E-08 | 8.14695 | 0.26994 | 1.51196 | 2 |
| **HSC** | 9.86E-08 | 10.44209 | 0.40610 | 1.92930 | 2 |
| **HSC** | 1.05E-07 | 9.81780 | 0.38179 | 1.81831 | 2 |
| **HSC** | 1.15E-07 | 6.98358 | 0.20361 | 1.31055 | 2 |
| **HSC** | 1.32E-07 | 10.43766 | 0.55596 | 1.91987 | 2 |
| **HSC** | 1.39E-07 | 7.35419 | 0.25496 | 1.38169 | 2 |
| **HSC** | 1.45E-07 | 8.72435 | 0.36878 | 1.62900 | 2 |
| **FIB** | 1.77E-05 | 5.97831 | 0.47317 | 1.33097 | 1 |
| **FIB** | 1.94E-05 | 5.76392 | 0.48322 | 1.28337 | 1 |
| **FIB** | 1.99E-05 | 5.64461 | 0.37723 | 1.28199 | 1 |
| **FIB** | 2.21E-05 | 6.50873 | 0.51902 | 1.46642 | 1 |
| **FIB** | 2.45E-05 | 6.37403 | 0.47630 | 1.45247 | 1 |
| **FIB** | 2.90E-05 | 6.62905 | 0.54404 | 1.51336 | 1 |
| **FIB** | 3.15E-05 | 5.97111 | 0.46286 | 1.37659 | 1 |
| **FIB** | 3.42E-05 | 5.89119 | 0.43623 | 1.37001 | 1 |
| **MSC** | 0.0003103 | 15.17442 | 1.62401 | 3.95927 | 3 |
| **MSC** | 0.0003112 | 15.40002 | 1.65415 | 4.01733 | 3 |
| **MSC** | 0.0003326 | 12.19501 | 1.01369 | 3.28523 | 3 |
| **MSC** | 0.0003413 | 16.56544 | 2.11651 | 4.25407 | 3 |
| **MSC** | 0.0003857 | 13.66328 | 1.33108 | 3.66721 | 3 |
| **MSC** | 0.0004143 | 15.01302 | 1.77504 | 3.95984 | 3 |

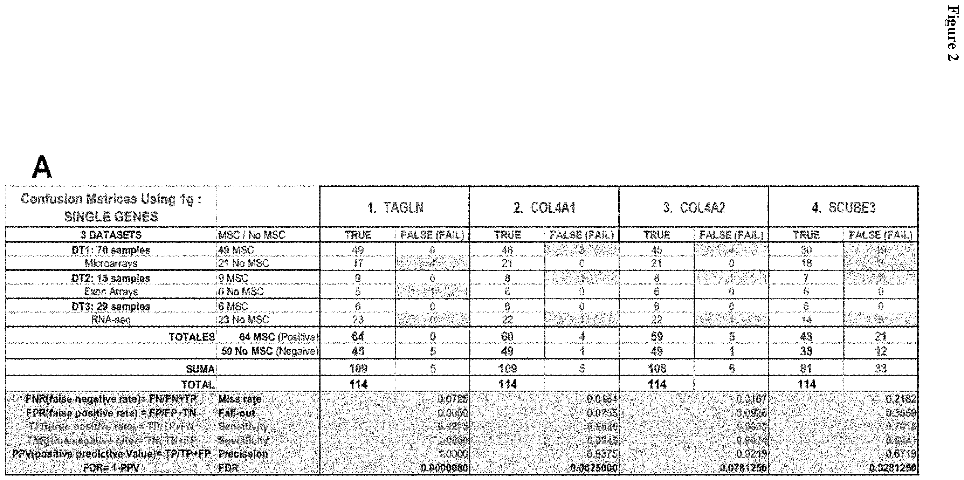

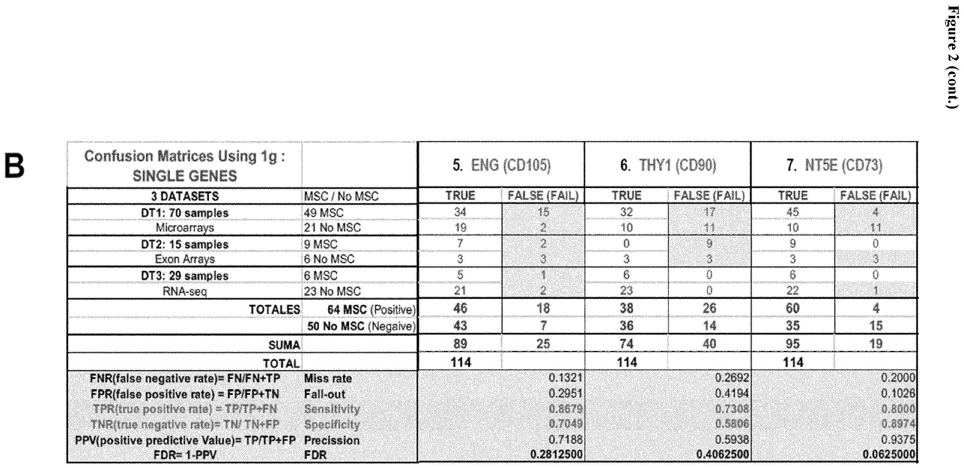

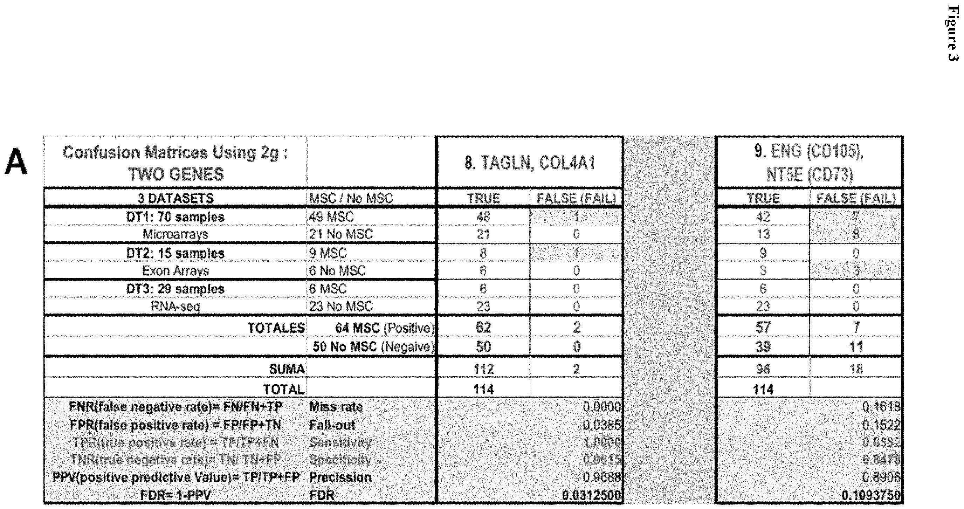

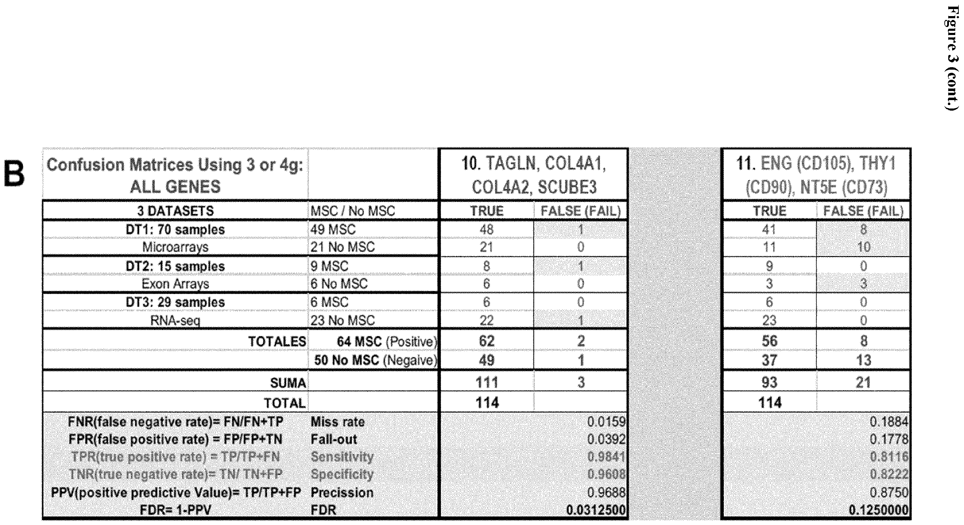

With all these numbers, since we know *α priori* the cell type of each sample, we can produce a confusion matrix and calculated all the statistical parameters derived from the errors: sensitivity, specificity, precision, miss-rate, fall-out and FDR p-value. All these statistical parameters are calculated for each one of the genes tested as candidate marker, and the results for each singular gene are presented in **Figure 2****.** Moreover, we also tested some combination of genes, as the 3 standard genes indicated above, and the statistical results of these tests are provided in **Figure 3****.** As a conclusion, we observed that TAGLN is the best gene marker of all the ones tested, presenting the best precision (value 1.0) and very good sensitivity=0.927 and specificity=1.0. Combined, TAGLN and COL4A1, are also very good markers of MSC **(****Figure 3****,** confusion matrix 8). In all cases these genes have less errors in the identification of MSC that the 3 standard genes (CD105, CD90 and CD73), that many times showed clear difficulties in the correct identification of FIB versus MSC.

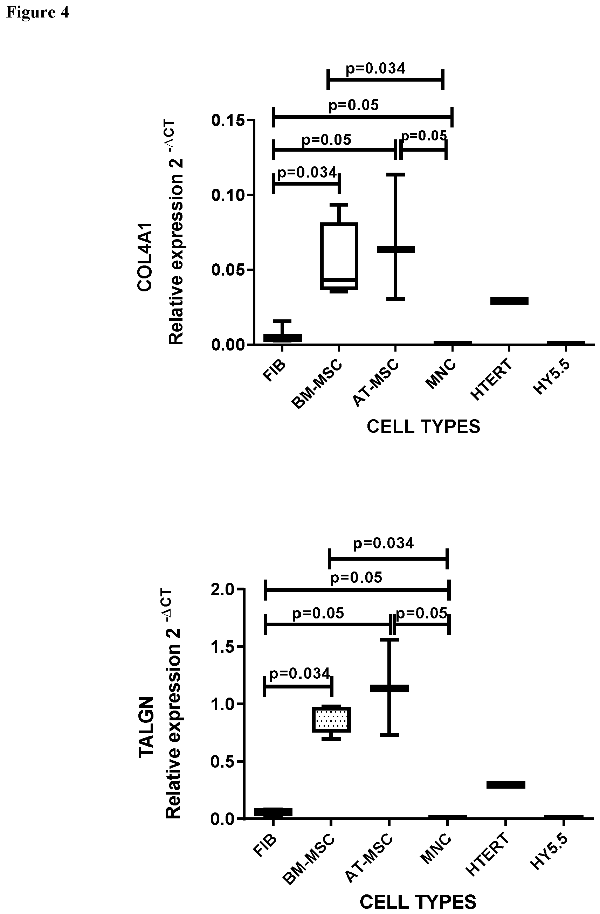

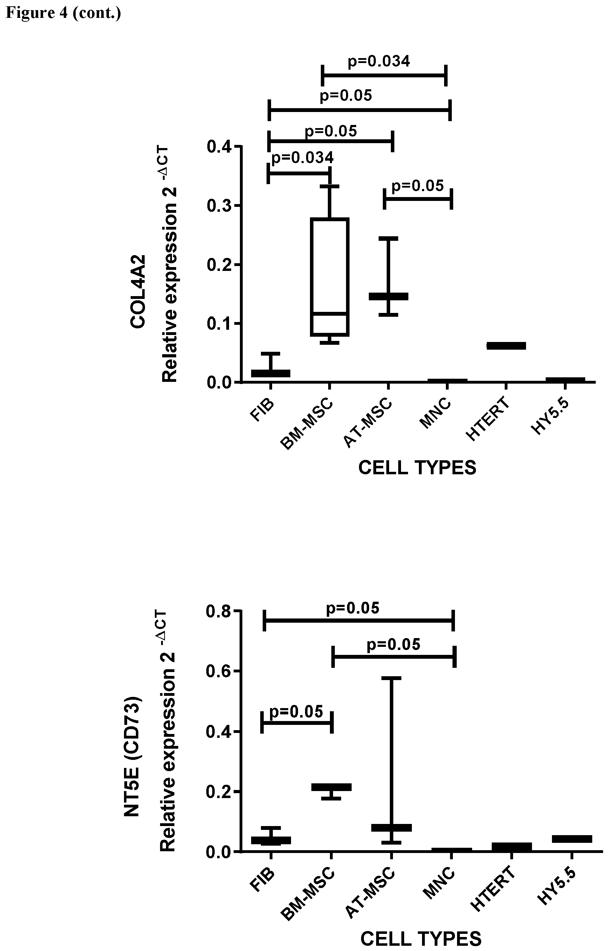

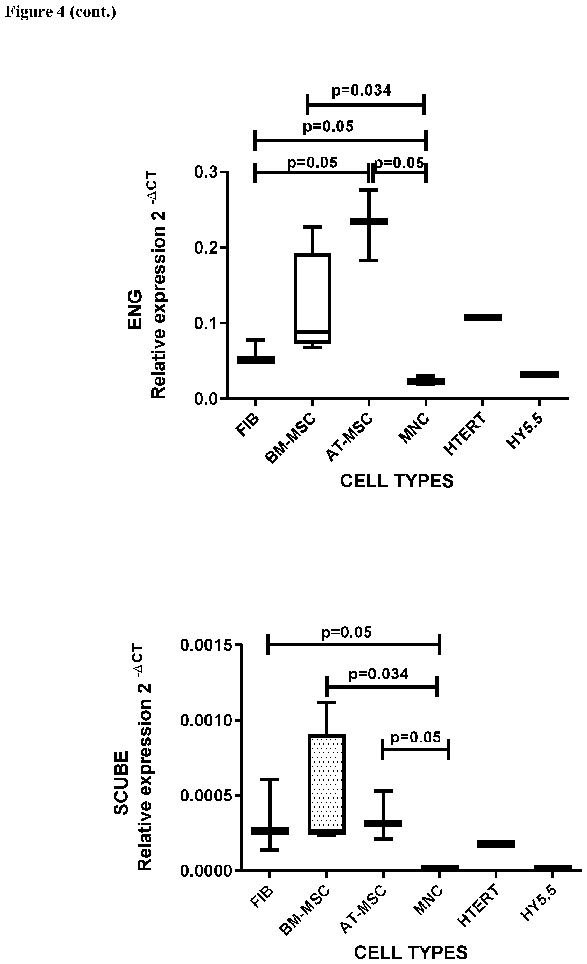

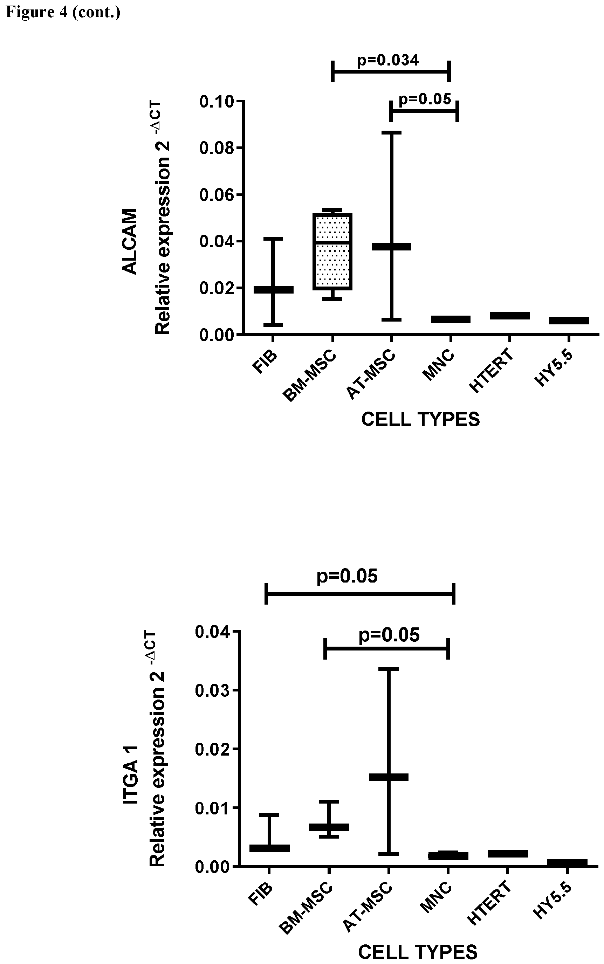

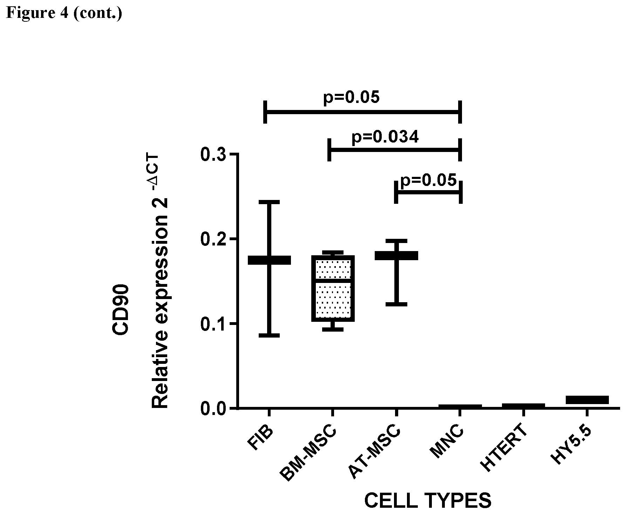

All these results were validated in an independent way using RT-PCR with other samples from 6 human cell types. The results are presented in **Figure 4****,** that shows the confirmation of the expression level of 9 genes tested in MSC from different sources (BM-MSC and AT-MSC), Fibroblasts (FIB), mononuclear cells (MNC) and two stromal cell lines (HTERT and HY5.5). The results indicated again that TAGLN, COL4A1 and COL4A2 are good and distinctive markers of MSC from bone marrow or adipose tissue, highly expressed in these cells and with very low expression in the other cells tested. In fact, these genes showed very significant differences between any type of MSC versus FIB. When comparing FIB vs BM-MSC the expression of NT5E (CD73) was also significantly higher in MSC from bone marrow (not so clear for AT-MSC) **(****Figure 4**). This gene marker, CD73, currently used as a standard to select MSC, was in fact the one that provided best results in the statistical analysis done with the Global Tests **(Figure 3B)**. Regarding the comparison between FIB and MSC from adipose tissue, in addition to TAGLN, COL4A1 and COL4A2, ENG expression is also differentially higher in MSC.

For the remaining genes tested, there were no differences between the expression in FIB and MSC (either BM-MSC or AT-MSC). It may be relevant to indicate that for the panel of 9 genes tested in this study, the mononuclear cells (MNC) isolated from peripheral blood did show very low not significant expression level when compared to the rest of studied cells. These cells, MNC, only showed a small signal for ALCAM (CD166) and for ITGA1 (CD49a) genes, but these genes (that are used in some studies to characterize MSC) are expressed in a similar level in FIB; therefore, they cannot be used as selective and distinct markers of MSC. In conclusion, our results confirm that the genes TAGLN, COL4A1 and COL4A2 can be used as specific markers of human MSC, showing clear differences with FIB. Out of these three genes, the one that provides best results in a consistent way is TAGLN (transgelin; Entrez Gene ID: 6876, Ensembl ID: ENSG00000149591, HGNC ID: 11553).

## Claims

1. *In vitro* method for identifying mesenchymal stem/stromal cells in a cell population obtained from a subject, which comprises assessing the expression of the gene TAGLN (Transgelin) in the cell population, wherein the expression of the gene TAGLN is an indication that the cells are mesenchymal stem/stromal cells.

2. *In vitro* method for differentiating mesenchymal stem/stromal cells from Fibroblasts in a cell population obtained from a subject, which comprises assessing the expression of the gene TAGLN, wherein the expression of the gene TAGLN is an indication that the cells are mesenchymal stem/stromal cells and that the cells are not Fibroblasts.

3. *In vitro* method, according to any of the previous claims, which further comprises assessing the expression of the gene COL4A1 (Collagen type IV alpha 1 chain) and/or COL4A2 (Collagen type IV alpha 2 chain), wherein the expression of the gene COL4A1 and/or COL4A2 is an indication that the cells are mesenchymal stem/stromal cells and that the cells are not Fibroblasts.

4. *In vitro* method, according to any of the previous claims, which comprises measuring the expression levels of the genes TAGLN, or [TAGLN and COL4A1], or [TAGLN and COL4A2], wherein if an overexpression of the genes is identified with respect to a pre-established threshold value determined in cells which are not mesenchymal stem/stromal cells, it is an indication that the cells are mesenchymal stem/stromal cells and that the cells are not Fibroblasts.

5. *In vitro* use of TAGLN for identifying mesenchymal stem/stromal cells, or for differentiating mesenchymal stem/stromal cells from Fibroblasts, in a cell population obtained from a subject.

6. *In vitro* use, according to claim 5, of [TAGLN and COL4A1], or [TAGLN and COL4A2], for identifying mesenchymal stem/stromal cells, or for differentiating mesenchymal stem/stromal cells from Fibroblasts, in a cell population obtained from a subj ect.

7. *In vitro* use of a kit comprising reagents for measuring the expression of TAGLN for identifying mesenchymal stem/stromal cells, or for differentiating mesenchymal stem/stromal cells from Fibroblasts, in a cell population obtained from a subject.

8. *In vitro* use, according to claim 7, of a kit comprising reagents for measuring the expression of [TAGLN and COL4A1], or [TAGLN and COL4A2], for identifying mesenchymal stem/stromal cells, or for differentiating mesenchymal stem/stromal cells from FIBroblasts, in a cell population obtained from a subject.

9. Kit consisting of reagents for measuring the expression of [TAGLN and COL4A1] or [TAGLN and COL4A2].

10. Substantially pure population of mesenchymal stem/stromal cells, wherein the mesenchymal stem/stromal cells are at least 80% of the total cell population, **characterized by** the expression of the gene TAGLN.

11. Substantially pure population of mesenchymal stem/stromal cells, according to claim 10, **characterized by** the expression of the genes [TAGLN and COL4A1] or [TAGLN and COL4A2].

12. Substantially pure population of mesenchymal stem/stromal cells, according to any of the claims 10 or 11, **characterized by** overexpressing of the genes TAGLN, or [TAGLN and COL4A1], or [TAGLN and COL4A2], with respect to a pre-established threshold value determined in cells which are not mesenchymal stem/stromal cells.

13. Pharmaceutical composition comprising the substantially pure population of mesenchymal stem/stromal cells of claims 10 to 12 and, optionally, pharmaceutically acceptable excipients or carriers.

14. Substantially pure population of mesenchymal stem/stromal cells according to any of the claims 10 to 12, or pharmaceutical composition according to claim 13, for use as a medicament.
